# EUROPÄISCHE PATENTSCHRIFT

(11) **EP 2 768 924 B1**
(45) Veröffentlichungstag und Bekanntmachung des Hinweises auf die Patenterteilung: **13.12.2017**
(21) Anmeldenummer: 12775219.4
(22) Anmeldetag: 12.10.2012
(51) Int. Cl.: C09K 8/584, C07C 41/03, C07C 43/23

(54) **VERWENDUNG VON TRIS(2-HYDROXYPHENYL)-METHAN-DERIVATEN FÜR DIE TERTIÄRE ERDÖLFÖRDERUNG**
USE OF TRIS(2-HYDROXYPHENYL)METHANE DERIVATIVES FOR TERTIARY PETROLEUM PRODUCTION
UTILISATION DE DÉRIVÉS DE TRIS(2-HYDROXYPHÉNYL)MÉTHANE POUR LA RÉCUPÉRATION TERTIAIRE DE PÉTROLE

(30) Priorität: 18.10.2011 EP 11185626
(43) Veröffentlichungstag der Anmeldung: 27.08.2014
(73) Patentinhaber: Wintershall Holding GmbH, 34119 Kassel (DE)
(72) Erfinder: OETTER, Guenter, 67227 Frankenthal (DE); HEINZ, Bjoern, 68159 Mannheim (DE); HANSCH, Markus, 67346 Speyer (DE); WEISS, Horst, 67141 Neuhofen (DE); DEGLMANN, Peter, 68163 Mannheim (DE); KURKAL-SIEBERT, Vandana, 69117 Heidelberg (DE); HEILMANN, Frank, 67246 Dirmstein (DE); AGLAVE, Ravindra, Katy, Texas 77494 (US); SIGGEL, Lorenz, 69121 Heidelberg (DE); WENZKE, Benjamin, 20251 Hamburg (DE)
(74) Vertreter: BASF IP Association
(86) Internationale Anmeldenummer: PCT/EP2012/070245
(87) Internationale Veröffentlichungsnummer: WO 2013/057044

(56) Entgegenhaltungen:
- EP-A1- 1 707 561
- WO-A1-2011/144643
- US-A1- 2007 107 897
- US-A1- 2011 288 322
- GECZY I ET AL: "SURFACE ACTIVE PROPERTIES AND POSSIBILITES OF TECHNOLOGICAL APPLICATION OF NON-IONIC ADDITION PRODUCTS OF POLYOXIETHYLATED FATTY ALCOHOLS WITH ISOCYANATES", KOLORISZTIKAI ÉRTESÍTO, K ZPONTI KOLORISZTIKAI KUTATÓ LABORATÓRIUM, BUDAPEST, HU, Bd. 24, Nr. 3, 1. Januar 1983 (1983-01-01) , Seiten 142-147, XP008050615, ISSN: 0023-2939
- CHEN X ET AL: "Micellization of and solute binding to amphiphilic poly(ethylene oxide) star polymers in aqueous media", LANGMUIR, AMERICAN CHEMICAL SOCIETY, NEW YORK, NY; US, Bd. 12, Nr. 9, 1. Januar 1996 (1996-01-01) , Seiten 2207-2213, XP002988265, ISSN: 0743-7463, DOI: 10.1021/LA950963P

## Beschreibung

Die vorliegende Erfindung betrifft die Verwendung von Tris(2-hydroxyphenyl)-methanDerivaten für die tertiäre Erdölförderung. Ein Gegenstand der Erfindung ist auch ein Verfahren für die Erdölförderung, bei dem man eine wässrige Formulierung einsetzt, die mindestens ein Tris(2-hydroxyphenyl)-methan-Derivat enthält, wobei diese durch mindestens eine Injektionsbohrung in eine Erdöllagerstätte, beispielsweise mit einer Lagerstätten-Temperatur von 10°C bis 150°C, eingepresst wird, und der Lagerstätte durch mindestens eine Produktionsbohrung das Rohöl entnimmt.

In natürlichen Erdölvorkommen liegt Erdöl oftmals in den Hohlräumen poröser Speichergesteine vor, welche zur Erdoberfläche hin von undurchlässigen Deckschichten abgeschlossen sind. Bei den Hohlräumen kann es sich um sehr feine Hohlräume, Kapillaren oder Poren handeln. Feine Porenhälse können beispielsweise einen Durchmesser von nur ca. 1 µm aufweisen. Neben Erdöl und Anteilen von Erdgas enthalten die Lagerstätten oftmals auch salzhaltiges Wasser. Insbesondere kann der Einsatz von Hilfsstoffen für die ErdölGewinnung aus Salz-reichen Gesteinsformationen schwierig sein.

Bei der Erdölförderung unterscheidet man zwischen der primären, sekundären und tertiären Förderung. Bei der primären Förderung strömt das Erdöl nach dem Anbohren der Lagerstätte aufgrund des Eigendrucks der Lagerstätte von selbst durch das Bohrloch an die Oberfläche. Der Eigendruck entsteht durch Auflast der im Wesentlichen wassergefüllten überlagerten Gesteinsschichten. Der Eigendruck der Lagerstätte lässt bei Entnahme von Erdöl aber oft schnell nach, so dass mittels der primären Förderung je nach Lagerstätte meist nur ca. 5 bis 10% der in der Lagerstätte vorhandenen Erdölmenge gefördert werden können. Danach reicht der Eigendruck nicht mehr zur Förderung von Erdöl aus, so dass danach oftmals Pumpen zur weiteren Erdölgewinnung eingesetzt werden.

Nach der primären Förderung kann daher die sekundäre Förderung zum Einsatz kommen, bei der zusätzlich zu den Bohrlöchern, welche der Förderung des Erdöls dienen, den sogenannten Produktionsbohrungen, weitere Bohrlöcher in die erdölführende Formation gebohrt werden. Durch diese sogenannten Injektionsbohrungen wird Wasser in die Lagerstätte eingepresst (das sogenannte "Wasserfluten"), um den Druck aufrechtzuerhalten oder wieder zu erhöhen.

Durch das Einpressen des Wassers bzw. einer entsprechenden wässrigen Formulierung wird das Erdöl außerdem durch die Hohlräume in der Formation langsam von der Injektionsbohrung ausgehend in Richtung der Produktionsbohrung gedrückt. Dies funktioniert so lange, wie das höher-viskose Öl durch das Wasser vor sich her geschoben wird. Sobald das dünnflüssige Wasser auf bevorzugten Fließwegen zu den Produktionsbohrungen durchbricht, strömt es ab diesem Zeitpunkt auf dem Weg des geringsten Widerstandes, also vor allem durch die gebildeten Fließwege und verdrängt kaum noch Öl. Mittels primärer und sekundärer Förderung sind im Regelfalle nur ca. 30 bis 35 % der in der Lagerstätte vorhandenen Erdölmenge zu fördern.

Nach den Maßnahmen der sekundären Erdölförderung (bzw. nach Maßnahmen der primären Erdölförderung) werden auch Maßnahmen der tertiären Erdölförderung (auch bekannt als "Enhanced Oil Recovery", EOR) zur weiteren Steigerung der Ölausbeute eingesetzt. Hierzu gehören Verfahren, bei denen man geeignete Chemikalien, wie Tenside und/oder Polymere als Hilfsmittel in Formulierungen zur Ölförderung einsetzt. Eine Übersicht zur tertiären Ölförderung unter Einsatz von Chemikalien findet sich beispielsweise in einem Artikel von D. G. Kessel von 1989 (Journal of Petroleum Science and Engineering, 2 (1989), 81 bis 101).

Zu den bekannten Techniken der tertiären Erdölförderung gehört auch das so genannte "Polymerfluten", bei dem man durch die Injektionsbohrungen eine wässrige Lösung eines verdickend wirkenden Polymers in die Erdöllagerstätte einpresst, wobei die Viskosität der wässrigen Polymerlösung an die Viskosität des Erdöls angepasst ist. Statt einer Polymerlösung können auch wässrige Lösungen mit nicht-polymeren Verdickern eingesetzt werden.

Verdicker sind Chemikalien, welche die Viskosität wässriger Lösungen bis hin zur Gelbildung erhöhen. Durch das Einpressen einer verdickten Lösung wird das Erdöl durch die Hohlräume in der Formation von der Injektionsbohrung ausgehend in Richtung der Produktionsbohrung gedrückt, und das Erdöl kann über die Produktionsbohrung gefördert werden. Dadurch, dass eine Verdicker-Formulierung etwa die gleiche Mobilität aufweist wie das Erdöl, wird die Gefahr verringert, dass die Formulierung ohne Effekt zur Produktionsbohrung durchbricht ("Fingering").

Somit kann mit Verdicker die Mobilisierung des Erdöls viel gleichmäßiger und effizienter erfolgen als bei der Verwendung von dünnflüssigem Wasser, indem das bei Verwendung von Wasser auftretende "Fingering" vermieden wird. Des Weiteren wird eine kolbenartige Verdrängung des Öls durch die Anpassung der Mobilität erreicht. Hierdurch wird die Förderung des mobilen Öls bezüglich des Wasserflutens beschleunigt.

Außerdem kommen bei der tertiären Erdölförderung auch Tenside zusätzlich zu Verdickern zum Einsatz. Tenside werden bei der Erdölförderung verwendet, um die Öl-Wasser Grenzflächenspannung auf sehr geringe Werte zu erniedrigen und somit weiteres Erdöl, das ansonsten im Gestein verbleiben würde, zu mobilisieren.

Durch das nachfolgende Einpressen einer verdickten Wasserlösung wird das so mobilisierte Erdöl wie beim Wasserfluten von der Injektionsbohrung ausgehend in Richtung der Produktionsbohrung gedrückt, und kann so über die Produktionsbohrung gefördert werden. Einzelheiten zum Fluten mit verdickten bzw. Tensid-haltigen Lösungen sowie hierzu geeigneter Komponenten werden beispielsweise beschrieben in "Petroleum, Enhanced Oil Recovery" (Kirk-Othmer, Encyclopedia of Chemical Technology, John Wiley & Sons, 2005).

Teilweise wird einer solchen Kombination von nacheinander geschaltetem "Tensidfluten" und "Polymerfluten" noch eine Phase mit einem alkalisches Agens wie Natronlauge vorgeschaltet, um im Rohöl vorhandene natürliche Tenside zu mobilisieren ("Alkalische Polymer-Tensidflutung"). Für die vorgenannte Kombination aus nacheinander geschaltetem "Tensidfluten" und "Polymerfluten" können auch sogenannte viskoelastische Tenside verwendet werden. Diese viskoelastischen Tenside sind grenzflächenaktive Substanzen, die in Lösung Assoziate bilden, die die Viskosität der Lösung erhöhen.

Für Beispiele hierzu wird verwiesen auf "Molecular Gels: Materials with Self-Assembled Fibrillar Networks" (Richard G. Weiss, Pierre Terech, Dec. 22, 2005), Advances in Colliod and Interface Science 128-130 (2006) 77-102). Mit viskoelastische Tensiden kann eine Verringerung der Grenzfläschenspannung erreicht werden, die mit polymeren Komponenten allein nicht erreicht werden kann. Verdickend wirkende Tenside für die Erdölförderung werden an verschiedenen Stellen beschrieben. In "Oilfield Reviews" (Vol. 16(4) (2004) 10-28) wird die Verwendung viskoelastischer Tensidsysteme als "fracturing fluids" beschrieben. Von V. Shvets wurde bereits 1985 die Stabilisierung von Suspensionen durch den Einsatz nicht-ionischer Tenside beschrieben (Journal of Applied Chemistry of USSR, 58 (6), 1985, 1220-1224).

Die nachfolgenden Dokumente US 2009/0056942, US 2007/0107897, US 2006/ 0183646, US 2006/0084579, US 2005/0155762, US 2009/291864, US 2006/105919, US 2004/214725, US 7,461,694, US 7,373,977, US 7,053,127, und GB-A 2,408,506 beschreiben die Anwendung viskoelastischer Tenside für Erdöl-Anwendungen, z.B. für die tertiäre Erdölförderung.

Die sogenannten Assoziate, die Tenside bilden können, auch Mizellen genannt, bilden sich aufgrund hydrophober Wechselwirkungen.

Die verdickenden Eigenschaften solcher Lösungen können in der Regel durch Scherung aufgehoben werden, bei der die Assoziate in kleinere Fragmente zerfallen. Bei diesem Prozess werden jedoch keine chemischen Bindungen gebrochen und die Assoziate bilden bei der Abwesenheit von Scherung ihre verdickende Wirkung wieder voll aus.

Hierin besteht ein Vorteil viskoelastischer Tensid-Systeme, insbesondere gegenüber synthetischen, polymeren Verdickern, die durch starke Scherung, wie sie beispielsweise beim Pumpen einer Lösung in ein Ölreservoir auftritt, irreversibel zerstört werden können. Hierzu kommt der Effekt, dass visko-elastische Tenside die Wasser-Öl-Grenzflächenspannung absenken, was bei Polymeren nicht oder nur in deutlich geringerem Ausmaß der Fall ist.

Eine Aufgabe der Erfindung ist es, ein Verfahren zur Erdölförderung bereitzustellen, das kostengünstig und technisch nicht aufwändig durchgeführt werden kann, und das auch bei salzhaltigen Ölvorkommen eingesetzt werden kann. Insbesondere bei hohen Salzgehalten (z. B. mindestens 10.000 ppm, oftmals mindestens 20.000 ppm, insbesondere mindestens 50.000 ppm) kann es von Vorteil sein, andere als polymere Verdicker auf synthetischer Basis bei der tertiären Erdölförderung einzusetzen.

Die erfindungsgemäß eingesetzten, nicht-polymeren Derivate von Tris(2-hydroxyphenyl)methanen, welche als funktionelle Gruppen Polyalkoxygruppen oder mit terminalen sauren Gruppen oder mit einem Rest mit mindestens 2 OH-Gruppen oder einer Aminoxidgruppe modifizierte Polyalkoxygruppen aufweisen, wirken als hochwirksame Tenside bzw. Verdicker, auch bei erhöhten Temperaturen und/oder hohem Salzgehalt.

Die Verbindung Tris(2-hydroxyphenyl)methan sowie verschiedene Derivate davon sind seit Jahren bekannt, so beschreibt G. Casiraghi (Tetrahedron Letters, No. 9, 679 bis 682, 1973) die Synthese von mono- oder dialkylierten Tris-(2-hydroxyphenyl)-methanen durch Umsetzung entsprechender Phenole mit Triethylorthoformaten. M. B. Dinger und M. J. Scott (Chem. Commun., 1999, 2525-2526, Inorg. Chem. 2000, 39, 1238-1254 sowie Inorg. Chem. 2001, 40, 1029-1036) beschreiben die Synthese verschiedener Tris-(3,5-dialkyl-2-hydroxyphenyl)-methan-Derivate, wobei als Alkylreste Methyl-, t-Butyl- und t-Pentyl-Reste beschrieben werden.

Die Trishydroxyphenylmethan-Verbindungen werden als Komplexbildner für Zink und Alkalimetallionen verwendet. M. B. Dinger und M. J. Scott, (Eur J. Org. Chem. 2000, 2467 bis 2478), beschreiben weiterhin die weitere Umsetzung der OH-Gruppe von Tris-(3,5-dialkyl-2-hydroxyphenyl)-methanen.

Die OH-Funktionen können durch Umsetzen mit Halogen-carbonsäureestern und Hydrolyse und/oder weiteren Umsetzungen derivatisiert werden. M. B. Dinger und Scott beschreiben beispielsweise Tris(3,5-di-t-butyl-2-carboxy-methoxyphenyl)methan, Tris(3,5-di-*tert-*butyl-2-[(dimethylamido)methoxy]-phenyl)-methan, Tris{3,5-di-*tert-*butyl-2-[*N-*(methylglycyl)-carbonylmethoxy]phenyl}-methan und Tris(3,5-di-*tert-*butyl-2-[(benzylamino-carbonyl)-methoxy]phenyl)-methan. Die Derivate können jeweils als Komplexbildner eingesetzt werden, beispielsweise für Zn(II)-Ionen.

Die Autoren K. Matloka (Dalton Trans., 2005, 3719 bis 3721 bzw. Separation Science and Technology, 41, 2006, 2129 bis 2146) sowie M. W. Peters (Inorg. Chem., 2002, 41, 1701 bis 1716) offenbaren funktionalisierte Tris-(3,5-dialkyl-2-hydroxyphenyl)-methane, und zwar tripodale Diglykolamide und deren Verwendung zur Komplexierung und Abtrennung von Lanthaniden. Als Zwischenstufe der Synthese werden Tris-(3,5-dialkyl-2-hydroxyphenyl)-methane verwendet, bei denen die OH-Gruppe mit ω-Amino- bzw. Cyanoalkylgruppen verethert ist. R. Mitra beschreibt (Dalton Trans., 2007, 3924 bis 3935) besondere Tris-(2-hydroxyphenyl)-methan-Derivate, welche terminale 2-Pyridylmethylpiperazin-Gruppen aufweisen. Diese Moleküle können Zinkionen binden und werden als Katalysatoren zur Phosphatdiester-Synthese verwendet. Als Zwischenstufe der mehrstufigen Synthese wird Tris-[2-(2-hydroxylethoxy)-3-methyl-5-t-butylphenyl]methan offenbart.

EP-A 0 597 806 offenbart Cyclohexylgruppen-haltige Glydidylether zur Verwendung als Reaktivverdünner, Flexibilisatoren oder Adhäsionsverbesserer. Als Zwischenprodukt der Synthese werden verschiedene Tris(2-hydroxyphenyl)methane beschrieben, darunter auch solche, bei denen die OH-Funktion mit einer (substituierten) 2-Hydroxyethylgruppe verethert ist. US 2009/0155714 offenbart Zusammensetzungen zur Herstellung von Photoresists. Als Komponente hierfür werden verschiedene Tris(2-hydroxyphenyl)methan-Derivate verwendet, bei denen die OH-Funktion jeweils mit verschiedenen Carbonsäuren verestert ist.

Es ist bekannt, dass Tenside oberhalb der kritischen Mizellbildungskonzentration (cmc) zu Mizellen aggregieren. Die Form dieser wasserlöslichen Aggregate hängt von der Struktur der Tenside sowie von äußeren Parametern wie Temperatur oder Elektrolytkonzentration ab. Typischerweise können sich oberhalb der Mizellbildungskonzentration sphärische oder stäbchenförmige Mizellen bilden. Die Charakterisierung von Mizellen kann mittels NMR-Spektroskopie erfolgen (siehe J.L. Lemyre in Langmuir 2010, 26 (9), 6250-6255). Bei bestimmten strukturellen Gegebenheiten und/oder äußeren Parametern können sich auch lange fadenförmige oder wurmartige Mizellen oder Assoziate bilden.
Als Folge davon kommt es bereits bei relativ niedriger Tensidkonzentration zu einer Verschlaufung und Überlappung dieser langen Aggregate, wodurch die Viskosität der Tensidlösung deutlich ansteigt. Eine bestimmte zeitliche Mindeststabilität der Mizellen ist dabei Voraussetzung. Dieses temporär gebildete Netzwerk aus Tensidmizellen reagiert aus rheologischer Sicht sowohl viskos als auch elastisch, weshalb man allgemein von viskoelastischen Tensidlösungen spricht.
Mizellen setzen einzelne Tenside frei, nehmen Tenside in den Mizellen-Verband auf, zerfallen und bilden sich wieder neu. Tensid-Mizellen, die viskoelastische Netzwerke ausbilden, sind zeitlich sehr stabil, bevor sie in einzelne Bruchstücke zerfallen und sich wieder neu bilden, so dass das mizellare Netzwerk einer Scherung der Tensidlösung Widerstand entgegensetzen kann und damit sowohl viskos als auch elastisch reagiert. Weitere Einzelheiten zu viskoelastischen, wurmförmige Mizellen bildenden Tensiden wie Hexadecyltrimethylammonium-p-toluolsulfonat oder Cetyl-pyridinium-salicylat sind beispielweise in H. Hoffmann et al. (Adv. Colloid Interface Sci. 1982, 17, 275 bis 298) oder M. R. Rojas (Journal of Colloid and Interface Science 342 (2010) 103 bis 109)) beschrieben.

Aufgrund der dargestellten Eigenschaften eignen sich viskoelastische Tenside ganz besonders als Verdicker und können in verschiedenen Bereichen der Technik eingesetzt werden.

US 2005/0155762 offenbart Betaine mit Alkylketten von 14 bis 24 C-Atomen, beispielsweise Oleyl-amidopropyl-betain oder Erucyl-amido-propyl-betain als verdickend wirkende, viskoelastische Tenside. Diese Betaine haben aber technische Nachteile, beispielsweise hinsichtlich ihrer Stabilität. US 7,461,694 offenbart zwitterionische Tenside mit Alkylketten von 16 bis 24 C-Atomen als viskoelastische Tenside.

WO 2008/100436 offenbart eine viskoelastische Tensidmischung aus kationischen, anionischen oder zwitterionischen Tensiden und einem Polymer. Die Tenside weisen Alkylkettenlängen von12 bis 25 C-Atomen auf.

In den zitierten Offenbarungen werden zur Bildung viskoelastischer Tensidlösungen jeweils Tenside mit langen Alkylketten eingesetzt. Ein Nachteil von viskoelastischen Tensiden mit langen Alkylketten ist, dass sie bei Kontakt mit unpolaren Flüssigkeiten diese solubilisieren, wodurch die wurmartigen Mizellen in sphärische Aggregate umgewandelt werden und die Viskoelastizität verloren geht. Außerdem bilden diese viskoelastischen Tenside in Kontakt mit anderen Tensiden in der Regel Mischmizellen, wodurch die Viskoelastizität ebenfalls verloren gehen kann.
Strukturen mit kurzen Alkylketten oder Strukturen, die vom üblichen linearen Bauprinzip der Tenside abweichen, bilden in der Regel sphärische Mizellen oder lediglich kurze anisometrische Aggregate und somit keine viskoelastischen Tensidlösungen.

Bekannte viskoelastischen Tenside sind oftmals kationische Tenside, wie Hexadecyltrimethylammonium-p-toluolsulfonat oder Cetylpyridiniumsalicylat. Kationische Tenside mit langen Alkylresten sind ökotoxikologisch bedenklich (siehe z.B. Versteeg et al., Chemosphere 24 (1992) 641). Da sie aufgrund ihrer positiven Ladung an Oberflächen besonders gut adsorbieren, verlieren sie darüber hinaus bei einigen Anwendungen an Wirkung. Daher besteht Bedarf nach Tensiden mit einem günstigeren ökotoxikologischem Profil und geringerer Adsorptionsneigung.

Überraschenderweise wurde nun gefunden, dass sich durch eine geeignete chemische Abwandlung von Tris(2-hydroxyphenyl)methan neuartige Wege und Verfahren zur Erdölgewinnung ergeben. Die Erfindung betrifft ein Verfahren zur Erdölförderung, bei dem man eine wässrige Formulierung, die mindestens ein Derivat von Tris-(2-hydroxyphenyl)-methan der allgemeinen Formel (I) enthält, durch mindestens eine Injektionsbohrung in eine Erdöllagerstätte einpresst und der Lagerstätte durch mindestens eine Produktionsbohrung das Rohöl entnimmt, 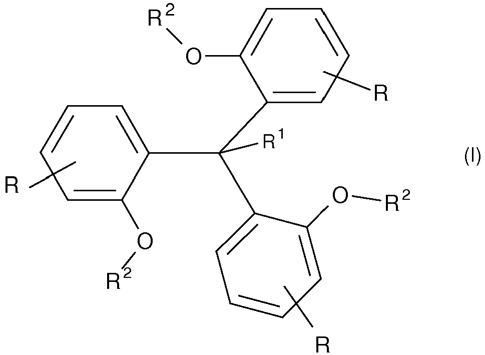 wobei in der Formel (I) die Reste R¹, R² und R die nachfolgende Bedeutung haben:
- R:: unabhängig voneinander jeweils 0 bis 4, vorzugsweise 1 bis 2, C₁- bis C₃₀-Kohlenwasserstoffreste pro Phenylring,
- R¹: ein Rest ausgewählt aus der Gruppe von H, OH, F, Cl, Br, I, sowie C₁- bis C₃₀-Kohlenwasserstoffgruppen, vorzugsweise H oder OH,
- R²: unabhängig voneinander Reste der allgemeinen Formel (III),

-(R⁵-O-)ₙ-R⁶-X (III)

wobei n für eine Zahl von 1 bis 50 steht, vorzugsweise 8 bis 35, und
wobei die Reste R⁵ unabhängig voneinander ausgewählt werden aus der Gruppe von Resten R⁷, R⁸ und R⁹, vorzugsweise aus der Gruppe R⁷ und R⁸, 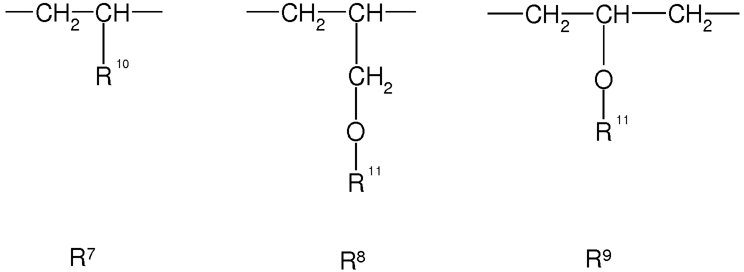 wobei R⁶, X, R¹⁰ und R¹¹ unabhängig voneinander die folgenden Bedeutungen haben:
- R⁶: eine Einfachbindung oder eine Alkylengruppe mit 1 bis 10 Kohlenstoffatomen, welche optional funktionelle Gruppen als Substituenten aufweisen kann,
- X: H oder eine saure Gruppe oder ein Rest mit mindestens 2 OH-Gruppen oder eine Aminoxidgruppe,
- R¹⁰: H oder ein C₁- bis C₆-Kohlenwasserstoffrest, vorzugsweise H oder Methyl
- R¹¹: eine Gruppe -(R⁵-O-)ₘ-R⁶-X, wobei m für eine Zahl von 0 bis 50 steht,
und wobei die Gesamtzahl z aller Gruppen R⁵ in einem Rest R² 1 bis 50, vorzugsweise 8 bis 35, beträgt, mit der Maßgabe, dass es sich bei der Zahl z um eine Zahl von 2 bis 50 handelt, falls mindestens ein X für H steht.

Diese Verbindungen sind chemisch und thermisch stabil, über einen langen Zeitraum lagerfähig und auch in Salz-haltigem Wasser bei höheren Temperaturen einsetzbar.

Die Erfindung betrifft auch ein Verfahren zur Erdölförderung, bei dem man eine wässrige Formulierung einsetzt, die neben Wasser mindestens ein Derivat von Tris-(2-hydroxyphenyl)-methan der allgemeinen Formel (II) enthält, 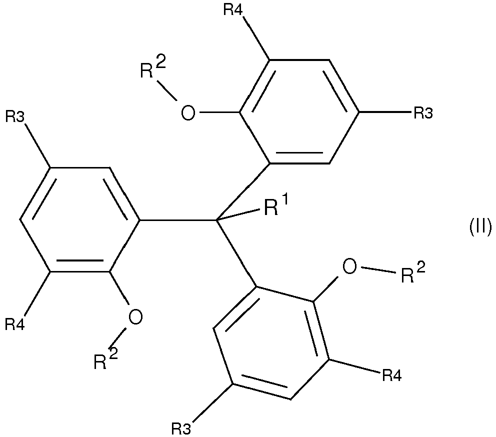 wobei R¹ und R² die oben genannten Bedeutungen haben und R³ und R⁴ unabhängig voneinander für H oder einen C₁- bis C₃₀-Kohlenwasserstoffrest, insbesondere für einen C₁-bis C₆-Kohlenwasserstoffrest, vorzugsweise einen verzweigten C4-Reste insbesondere t-Butylrest stehen.

Die Erfindung betrifft auch ein Verfahren, bei dem man eine Verbindung der Formel (II) eingesetzt wird, in der alle R³ und R⁴ unabhängig voneinander gerad-kettige oder verzweigte aliphatische C₁- bis C₆- Kohlenwasserstoffreste sind, insbesondere t-Butylreste.

Die Erfindung betrifft auch ein Verfahren, bei dem die saure Gruppe ausgewählt wird aus Carboxylgruppen -COOM, Sulfonsäuregruppen -SO₃M Sulfatgruppen -OSO₃M, Phosphonsäure-gruppen -PO₂M₂ oder Phosphorsäuregruppen -OPO₃M₂, wobei M für H⁺ oder ein einwertiges Gegenion, insbesondere Na-, K- oder Ammonium-Ionen, steht.

Die Erfindung betrifft auch ein Verfahren, wobei es sich bei den Resten R⁵ um Gruppen R⁷ und/oder R⁸ handelt.

Die Erfindung betrifft auch ein Verfahren, bei dem die eingesetzten Derivate von Tris-(2-hydroxyphenyl)-methan eine mittlere Molmasse von 2.000 bis 3.000 g/mol aufweisen und dass die drei (2-Hydroxyphenyl)-Gruppen gleiche Strukturen haben.
Die Erfindung betrifft auch ein Verfahren, bei dem die Temperatur der Erdöllagerstätte 10 bis 150°C, insbesondere 10 bis 120°C, oftmals auch 10 bis 90°C beträgt.

Die Erfindung betrifft auch ein Verfahren, bei dem die wässrige Formulierung als weitere Komponente mindestens ein Salz in einer Menge von 10.000 ppm bis 350.000 ppm enthält.

Die Erfindung betrifft auch ein Verfahren, bei dem die Erdölförderung aus Lagerstätten mit sehr hohem Salzgehalten und einer Lagerstätten-Temperatur von 10° bis 150°C erfolgt, wobei die Lagerstätte neben Erdöl auch Lagerstätten-Wasser mit einer Salinität von 10.000 ppm bis 350.000 ppm, oftmals 20.000 ppm bis 350.000 ppm, insbesondere von 100.000 ppm bis 350.000 ppm umfasst, und das Erdöl eine Viskosität (gemessen bei Lagerstätten-Temperatur) von mindestens 3 mPa*s aufweist, indem man eine wässrige Formulierung umfassend mindestens ein Derivat von Tris-(2-hydroxyphenyl)-methan der Formel (I) oder der Formel (II) durch mindestens eine Injektionsbohrung in die Erdöllagerstätte einpresst und der Lagerstätte durch mindestens eine Produktionsbohrung Rohöl entnimmt, wobei das Verfahren mindestens die folgenden Verfahrensschritte umfasst:
- Bereitstellen mindestens eines Tris-(2-hydroxyphenyl)-methan-Derivats der allgemeinen Formel (I) oder (II) als Rein-Substanz, Mischung oder als Konzentrat;
- Herstellen der wässrigen Formulierung der Tris-(2-hydroxyphenyl)-methan-Komponente(n) durch Verdünnen des gemäß Schritt (1) bereitgestellten Konzentrats (K) vor Ort mit Wasser auf eine Konzentration von 0,01 g/l bis 10 g/l, vorzugsweise sowie von 0,05 g/l bis 5 g/l,
- Injizieren der wässrigen Formulierung der Tris-(2-hydroxyphenyl)-methan-Komponente(n) in die Erdölformation, und
- Entnahme von Rohöl durch mindestens eine Produktionsbohrung.

Die Erfindung betrifft auch ein Verfahren, wobei es sich bei dem (mindestens einem) eingesetzten Derivat von Tris-(2-hydroxyphenyl)-methan um ein unverzweigtes Derivat handelt und die Konzentration des Tris(2-hydroxyphenyl)methan-Derivats in der Formulierung von 0,01 g/l bis 10 g/l, vorzugsweise 0,05 g/l bis 5 g/l beträgt.
Ein Gegenstand der Erfindung ist auch die Verwendung eines Tris(2-hydroxyphenyl)methan-Derivats der Formel (I) oder der Formel (II) wie oben beschrieben als Verdicker, insbesondere als visko-elastischer Verdicker, in einer wässrigen Formulierung für die Erdölförderung.

Die Erfindung betrifft auch eine wässrige Formulierung (z. B. als Konzentrat oder gebrauchsfertige Zusammensetzung), als Komponenten enthaltend:
0,001 bis 99 Gew. % mindestens eines Tris(2-hydroxyphenyl)methan-Derivats der Formel (I) gemäß Anspruch 1,
10 000 ppm bis 350 000 ppm anorganisches Salz, sowie ggf.
0,01 bis 5 Gew. % weiterer Hilfskomponenten für die Erdölförderung, sowie Wasser und ggf. weitere Lösungsmittel.

Die Erfindung betrifft auch eine wässrige Formulierung, enthaltend:
0,1 bis 90 Gew. % eines Tris(2-hydroxyphenyl)methan-Derivats der Formel (II)
10 000 ppm bis 350 000 ppm, oftmals 50 000 ppm bis 350 000 ppm anorganisches Salz, sowie
0,01 bis 5 Gew. % mindestens einer Hilfskomponente aus der Gruppe Tenside, Antioxidantien und Biozide, sowie Wasser und ggf. weitere Lösungsmittel.

Zum Fluten der Erdöl-Lagerstätte wird eine wässrige Lösung des Derivats (I) durch eine Bohrung (die sogenannte Injektionsbohrung) in eine Erdöllagerstätte verpresst, wobei die Mobilität der Lösung unter Formationsbedingungen etwa der Mobilität des Erdöls entsprechen sollte. Geeignete Verdicker für das Fluten müssen daher die verdickende Wirkung auch unter den Bedingungen der Erdöllagerstätte aufweisen, also auch bei Temperaturen oberhalb der Raumtemperatur sowie in Gegenwart von stark salzhaltigem Formationswasser. Formationswässer können bis zu 35 Gew. % Salze enthalten. Bei den Salzen handelt es sich beispielsweise um Alkalimetallsalze aber auch Erdalkalimetallsalze. Formationstemperaturen können bis zu 150°C betragen.

Eine weitere Aufgabe der Erfindung ist es, ein Verfahren zum Fluten von Erdölvorkommen bereitzustellen, mit dem auch bei höheren Formationstemperaturen und/oder hohen Salzgehalten zufriedenstellende Ergebnisse erzielt werden.
Dementsprechend wurde ein Verfahren zur Erdölförderung gefunden, bei dem man eine wässrige Formulierung umfassend mindestens ein Tris(2-hydroxyphenyl)-methan-Derivat durch mindestens eine Injektionsbohrung in eine Erdöllagerstätte einpresst und der Lagerstätte durch mindestens eine Produktionsbohrung Rohöl entnimmt.

Die Menge des Tris(2-hydroxyphenyl)-methan-Derivats in der Formulierung beträgt vorzugsweise 0,01 bis 10 Gew. %, oftmals auch 0,05 bis 10 Gew. % (bezogen auf die gesamte Formulierung).
In einer weiterhin bevorzugten Ausführungsform umfasst die wässrige Formulierung weiterhin Salze in einer Menge von 10 000 ppm bis 350 000 ppm.

Überraschenderweise wurde gefunden, dass sich die Viskosität der für das Verfahren verwendeten wässrigen Formulierungen mit steigender Temperatur nur wenig verändert. Auch bei höheren Temperaturen (z. B. 75° C) und in Salz-reichen Lösungen (z. B. größer 30 000 ppm) kann eine Ausfällung des Tris(2-hydroxyphenyl)-methan-Derivats (Trübung) vermieden werden.

Es wird ein besonders gutes Verhältnis von erzielter Viskosität zu eingesetzter Stoffmenge an Tris(2-hydroxyphenyl)-methan-Derivat (I) erreicht.

Die Erfindung kann wie folgt präzisiert werden:

### Eingesetzte Tris(2-hydroxyphenyl)-methan-Derivate

Für das erfindungsgemäße Verfahren zur Erdölförderung wird eine wässrige Formulierung mindestens eines Tris(2-hydroxyphenyl)-methan-Derivats eingesetzt und durch eine Injektionsbohrung in eine Erdöllagerstätte injiziert.

Bei den erfindungsgemäß verwendeten Verbindungen handelt es sich um Derivate von Tris(2-hydroxyphenyl)methan der allgemeinen Formel (I).

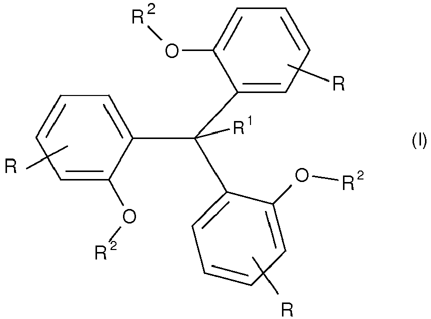

Bei dem Rest R¹ handelt es sich um einen Rest ausgewählt aus der Gruppe von H, OH, F, Cl, Br, I, oder geradkettigen, verzweigten oder cyclischen, aliphatischen und/oder aromatischen C₁- bis C₃₀-Kohlenwasserstoffgruppen. Bevorzugt handelt es sich um H, Cl, eine geradkettige oder verzweigte C₁- bis C₁₂-Alkylgruppe oder eine Benzylgruppe. Besonders bevorzugt handelt es sich bei R¹ um H.

Die drei Phenylringe können jeweils in 3, 4, 5 und 6-Stellung unabhängig voneinander mit Kohlenwasserstoffresten R mit 1 bis 30 Kohlenstoffatomen substituiert sein, wobei die Gruppen beliebig angeordnet sein können. Bevorzugt handelt es sich um 1 oder 2 Gruppen R pro Phenylring. Bei den Gruppen R kann es sich um geradkettige, verzweigte oder cyclische, aliphatische und/oder aromatische Kohlenwasserstoffreste handeln. Bevorzugt handelt es sich bevorzugt um geradkettigte, verzweigte oder cyclische aliphatische Kohlenwasserstoffgruppen mit 1 bis 20, besonders bevorzugt 1 bis 12 Kohlenstoffatomen. Beispiele geeigneter Gruppen R umfassen Methyl-, Ethyl-, n-Propyl-, Isopropyl-, n-Butyl-, i-Propyl-, t-Butyl, n-Pentyl-, 1,1-Dimethylpropyl-, 2,2-Dimethylpropyl, 3-Methylbutyl-, Hexyl-, 2-Ethylhexyl, Heptyl-, Octyl-, Nonyl-, Decyl-, Undecyl-, Dodecyl-, Cyclopentyl-, Cyclohexyl-, Adamantyl- oder Benzylgruppen. Die Gruppe tert-Butyl ist besonders geeignet.

Bevorzugt weisen die erfindungsgemäß eingesetzten Verbindungen die allgemeine Formel (II) auf.

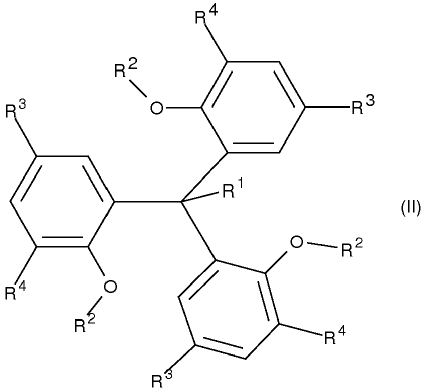

In Formel (II) stehen R³ und R⁴ jeweils unabhängig voneinander für H oder Kohlenwasserstoffreste mit 1 bis 30 C-Atomen, bevorzugt 1 bis 20 C-Atomen, besonders bevorzugt 1 bis 12 C-Atomen. Die Kohlenwasserstoffreste können geradkettig, verzweigt, cyclisch, aliphatisch und/oder aromatisch sein. Bevorzugt handelt es sich um geradkettige, verzweigte oder cyclische aliphatische Kohlenwasserstoffgruppen mit 1 bis 20, besonders bevorzugt 1 bis 12 Kohlenstoffatomen und ganz besonders bevorzugt um geradkettige oder verzweigte aliphatische Kohlenwasserstoffreste mit 1 bis 6 Kohlenstoffatomen.

Beispiele geeigneter Kohlenwaserstoffgruppen umfassen Methyl-, Ethyl-, Propyl-, Isopropyl-, n-Butyl-, i-Butyl, t-Butyl, n-Pentyl-, 1,1-Dimethylpropyl-, 2,2-Dimethylpropyl, 3-Methylbutyl-, Hexyl-, 2-Ethylhexyl, Heptyl-, Octyl-, Nonyl-, Decyl-, Undecyl-, Dodecyl-, Cyclopentyl-, Cyclohexyl- oder Adamantylgruppen. Bevorzugt handelt es sich bei den Resten R³ und R⁴ um H oder Methyl-, Ethyl-, Propyl-, Isopropyl-, n-Butyl-, i-Butyl, t-Butyl, n-Pentyl-, 1,1-Dimethylpropyl-, 2,2-Dimethylpropyl, 3-Methylbutylgruppen, 1,1,3,3-Tetramethylbutyl, besonders bevorzugt um t-Butylgruppen.

In einer bevorzugten Ausführungsform der Erfindung ist mindestens einer der Reste R³ oder R⁴ nicht H, besonders bevorzugt ist bei dieser Ausführungsform der Rest R³ nicht H. Ganz besonders bevorzugt sind beide Reste R³ und R⁴ jeweils nicht H. Bevorzugte, besonders bevorzugte und ganz besonders bevorzugte Kombinationen von Resten R³ und R⁴ sind in den nachfolgenden Tabellen 1, 2 und 3 genannt:

**Tabelle 1: Liste bevorzugter Kombinationen von Substituenten**

| R³ | R⁴ |
|---|---|
| t-Butyl | H |
| t-Butyl | Methyl |
| t-Butyl | Ethyl |
| t-Butyl | t-Butyl |
| Me | Me |
| Me | tBu |
| 1,1-Dimethylpropyl | H |
| 1,1-Dimethylpropyl | Methyl |
| 1,1-Dimethylpropyl | Ethyl |
| 1,1-Dimethylpropyl | t-Butyl |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

**Tabelle 2: Liste bevorzugter Kombinationen von Substituenten**

| R³ | R⁴ |
|---|---|
| t-Butyl | Methyl |
| t-Butyl | t-Butyl |
| 1,1-Dimethylpropyl | Methyl |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

**Tabelle 3: Liste bevorzugter Kombinationen von Substituenten**

| R³ | R⁴ |
|---|---|
| t-Butyl | t-Butyl |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

Ganz besonders bevorzugt handelt es sich sowohl bei R³ als auch bei R⁴ um tert.-Butylreste.

Bei den Resten R² in den oben genannten Formeln (I) und (II) handelt es sich unabhängig voneinander um Reste der allgemeinen Formel

-(R⁵-O-)ₙ-R⁶-X (III).

Bei den Resten R⁵ in Formel (III) handelt es sich unabhängig voneinander um Gruppen ausgewählt aus der Gruppe von Resten R⁷, R⁸ und R⁹

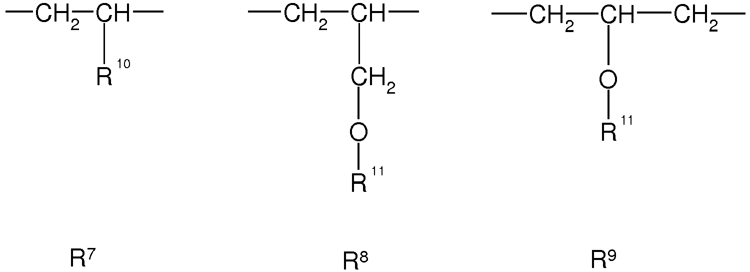

Hierbei stehen in den Gruppen R⁷ die Reste R¹⁰ unabhängig voneinander für H oder einen Kohlenwasserstoffrest mit 1 bis 6 Kohlenstoffatomen, bevorzugt einen aliphatischen Kohlenwasserstoffrest mit 1 bis 6 Kohlenstoffatomen. Anstelle der in Formel (III) dargestellten Orientierung -CH₂-CH(R¹⁰)- kann die Alkylengruppe auch in inverser Orientierung - CH(R¹⁰)-CH₂- in die Polyoxyalkylenkette eingebaut sein. Die Formel (III) soll beide Orientierungen umfassen, wobei selbstverständlich auch beide Orientierungen in einer Ketten enthalten sein können. Beispiele für Reste R¹⁰ umfassen H sowie Methyl-, Ethyl-, n-Propyl oder Phenylreste. Insbesondere handelt es sich bei R¹⁰ um H, Methyl-, Ethyl- oder n-Propylreste, bevorzugt um H, Methyl- oder Ethylreste, besonders bevorzugt um H oder Methyl.

In den Gruppen R⁸ und R⁹ stehen die Gruppen R¹¹ für Gruppen der allgemeinen Formel - (R⁵-O-)ₘ-R⁶-X, wobei m für eine Zahl von 0 bis 50 steht. Es kann sich also (für den Fall m = 0) um eine Gruppe -R⁶-X handeln, welche direkt mit dem O-Atom verknüpft ist, oder (für den Fall m > 0) um eine Gruppe, bei der -R⁶-X über eine Polyalkoxygruppe mit dem O-Atom verknüpft ist. Aufgrund der Tatsache, dass in letzterem Falle R¹¹ selbst wieder Gruppen R⁸ und R⁹ enthalten kann, können die Gruppen R² auch mehrfach verzweigt sein.

Die Reste R⁷, R⁸ und R⁹ können beliebig in der Resten R² angeordnet werden, beispielsweise statistisch, blockweise, alternierend oder mit einem Gradienten.
Bevorzugt eingesetzte Verbindungen der Formel (I) bzw. (II) sind Strukturen mit reinen Ethylenoxid-Ketten mit mittleren Kettenlängen zwischen 6-15 EO-Einheiten. Besonders bevorzugte sind auch EO-Glycidol-Copolymere mit 6-12 EO Einheiten und 1-5 Glycidol-Einheiten. Von diesen sind Copolymere mit einer Block-Copolymerstruktur besonders geeignet.

In den obigen Resten steht X jeweils für H oder eine saure Gruppe oder einen Rest mit mindestens 2 OH-Gruppen oder eine Aminoxidgruppe. Bei sauren Gruppen handelt es sich bevorzugt um eine Gruppe ausgewählt aus der Gruppe von Carboxylgruppen -COOM, Sulfonsäuregruppen -SO₃M, Sulfatgruppen -OSO₃M, Phosphonsäuregruppen -PO₂M₂ oder Phoshorsäuregruppen -OPO₃M₂, für wobei M für H⁺ oder ein einwertiges Gegenion steht. Die sauren Gruppen können also als freie Säure vorliegen und/oder als ein Salz davon. Sofern es sich bei M nicht um H⁺ handelt, handelt es sich bevorzugt um ein einwertiges Gegenion, wie beispielsweise NH₄⁺-, Ammoniumionen mit organischen Resten oder Alkalimetallionen. Bevorzugte saure Gruppen sind solche ausgewählt aus der Gruppe von Carboxylgruppen -COOM, Sulfonsäuregruppen -SO₃M oder Sulfatgruppen -OSO₃M, besonders bevorzugt Sulfatgruppen -OSO₃M.

Bevorzugte Gruppen mit mindestens 2 OH-Gruppen umfassen insbesondere Mono- oder Oligosaccharidreste, bevorzugt Monosaccharidreste. Es kann sich prinzipiell um alle Arten von Sacchariden handeln. Bevorzugt können von Pentosen und Hexosen, insbesondere von Hexosen abgeleitete Reste eingesetzt werden. Beispiele geeigneter Monosaccharide umfassen Glucose, Mannose, Galaktose, Fruktose oder Ribose. Bevorzugt können von Glukose abgeleitete Rest eingesetzt werden. Es kann sich auch um Derivate der Saccharide handeln, beispielsweise um durch Reduktion oder Oxidation aus den Sacchariden hervorgehende Produkte. Insbesondere kann es sich bei derartigen Derivaten um Zuckersäuren wie beispielsweise Gluconsäure handeln.

Je nach Art des Restes R², können nur eine Gruppe X oder auch mehrere Gruppen X in einem Rest R² enthalten sein.

Bei R⁶ handelt es sich um eine Einfachbindung oder eine Alkylengrupe mit 1 bis 10 Kohlenstoffatomen, bevorzugt 1 bis 3 Kohlenstoffatomen, welche optional funktionelle Gruppen als Substituenten, insbesondere eine OH-Gruppe aufweisen kann. Beispiele derartiger Gruppen umfassen -CH₂-, -CH₂CH₂-, -CH₂-CH₂-CH₂- oder -CH₂-CH(OH)-CH₂-Gruppen.

Die Gesamtzahl aller Gruppen R⁵ in einem Rest R², d.h. der Gruppen R⁵ in der Hauptgruppe als auch der Gruppen R⁵ in eventuell vorhandenen Verzweigungen, soll nachfolgend mit z bezeichnet werden. Für den Fall, dass es sich um eine lineare Gruppe R² handelt, entspricht z der Zahl n.
Erfindungsgemäß handelt es sich bei z um eine Zahl von 2 bis 50 für den Fall, dass mindestens ein X für H steht, und es handelt sich um eine Zahl von 1 bis 50 für den Fall, dass kein X für H steht. Die Zahlen z, n und m beziehen sich dabei in bekannter Art und Weise auf den Mittelwert der im Molekül vorhandenen Alkoxygruppen, wobei der Mittelwert selbstverständlich nicht eine natürliche Zahl sein muss, sondern auch eine positive rationale Zahl sein kann. In der Regel steht z unabhängig von der Natur des Substituenten X für eine Zahl von 2 bis 50, insbesondere 4 bis 40, bevorzugt 5 bis 30, besonders bevorzugt 8 bis 20 und ganz besonders bevorzugt 10 bis 15.

Der Fachmann trifft unter den möglichen Gruppen (III) sowie der Reste R¹, R², R³ und R⁴ je nach dem gewünschten Einsatzzweck der Verbindungen bei der Erdölförderung eine entsprechende Auswahl.

In einer ersten bevorzugten Ausführungsform der Erfindung handelt es sich bei mindestens einem der Reste R⁵ um einen Rest R⁷. In einer besonders bevorzugten Ausführungsform der Erfindung handelt es sich bei allen Resten R⁵ um Reste R⁷. Sofern die Reste R⁷ verschiedene Reste R¹⁰ umfassen, können diese auf beliebige Art und Weise angeordnet werden, beispielsweise statistisch, blockweise, alternierend oder mit einem Gradienten. Bevorzugt handelt es sich zumindest bei einem Teil der z Reste R¹⁰ um H, insbesondere bei mindestens 20 %, bevorzugt bei mindestens 50 %, besonders bevorzugt bei mindestens 80 % und ganz besonders bevorzugt handelt es sich bei allen Resten R¹⁰ um H.

In einer bevorzugten Ausführungsform der Erfindung handelt es sich bei den Resten R² um Gruppen -(R⁷-O-)ₙ-R⁶-X (IIIa). Reste R⁸ und Reste R⁹ sind bei dieser Ausführungsform nicht vorhanden.
Besonders bevorzugt handelt es sich um Gruppen -(R⁷-O-)ₙ-H (IIIb), d.h. X = H und R⁶ ist eine Einfachbindung.

In (IIIa) und (IIIb) steht n für eine Zahl 5 bis 30, besonders bevorzugt 8 bis 20 und ganz besonders bevorzugt 10 bis 15. Bei den Resten R¹⁰ in den Resten R⁷, d.h. den Resten - CH₂-CH(R¹⁰)-, handelt es sich unabhängig voneinander um einen Rest ausgewählt aus der Gruppe von H, Methyl oder Ethyl, wobei es sich bei mindestens 50 % der Reste (R¹⁰) um H handelt, bevorzugt bei mindestens 80 % der Reste und ganz besonders bevorzugt handelt es sich bei allen Resten R¹⁰ um H. Sofern mehrere verschiedene Reste R¹⁰ vorhanden sind, können diese statistisch, alternierend oder blockweise angeordnet sein. Im Falle von blockweiser Anordnung ist es bevorzugt, wenn die Ethylenoxidgruppen (also R¹⁰ = H) den terminalen Block bilden.

In einer weiteren bevorzugten Ausführungsform der Erfindung handelt es sich bei den Resten R² um Gruppen -(--CH₂-CH(R¹²)-O-)ₐ-(--CH₂-CH₂-O-)_{b}-H (IIIc), wobei die beiden Blöcke in dieser Reihenfolge angeordnet sind. Es handelt sich also wiederum um eine Ausführungsform, in der nur Gruppen R⁷ vorhanden sind, X ist H und R⁶ ist eine Einfachbindung.

Bei den Resten R¹² in der Formel (IIIc) handelt es sich um Kohlenwasserstoffreste mit 1 bis 6 Kohlenstoffatomen, bevorzugt aliphatische Kohlenwasserstoffreste mit 1 bis 6 Kohlenstoffatomen, bevorzugt eine Methyl und/oder eine Ethylgruppe. Bei a handelt es sich um Zahlen von 1 bis 49, bevorzugt 1 bis 10 und bei b um Zahlen von 1 bis 49, bevorzugt 1 bis 20 mit der Maßgabe, dass a + b = n = 2 bis 50. Bevorzugt ist b ≥ a.

In einer weiteren bevorzugten Ausführungsform der Erfindung handelt es sich bei den Resten R² um Gruppen -(-CH₂-CH(R¹³)-O-)ₙ-R⁶-X (IIId), wobei bei mindestens einer der Gruppen R² der Rest X nicht für H steht. Bei den Resten R¹⁰ in der Formel (IIId) handelt es sich unabhängig voneinander um einen Rest ausgewählt aus der Gruppe von H, Methyl oder Ethyl, wobei es sich bei mindestens 20 % der Reste R¹⁰ um H handelt, bevorzugt bei mindestens 50 % der Reste, besonders bevorzugt bei mindestens 80 % der Reste und besonders bevorzugt handelt es sich bei allen Resten R¹⁰ um H. Sofern mehrere verschiedene Reste R¹³ vorhanden sind, können diese statistisch, alternierend oder blockweise angeordnet sein. Im Falle von blockweiser Anordnung ist es bevorzugt, wenn die Ethylenoxidgruppen (also R¹³ = H) den terminalen Block bilden. Bevorzugt handelt es sich bei X in Formel (IIIc) um Carboxylgruppen -COOM, Sulfonsäuregruppen -SO₃M, Sulfatgruppen -OSO₃M. Beispielsweise kann es sich um Gruppen -(-CH₂-CH(R¹³)-O-)ₙ-H in Kombination mit Gruppen -(-CH₂-CH(R¹³)-O-)ₙ-SO₃H handeln. Bei n handelt es sich in dieser Ausführungsform bevorzugt um Zahlen von n = 1 bis 30.

In einer weiteren bevorzugten Ausführungsform der Erfindung umfassen die Reste R² der allgemeinen Formel -(R⁵-O-)ₙ-R⁶-X (III) Reste R⁸ und/oder R⁹. Es kann sich sowohl um Reste handeln, welche ausschließlich Gruppen R⁸ und/oder R⁹ umfassen, als auch um solche, welche neben R⁸ und/oder R⁹ zusätzlich Gruppen R⁷ umfassen. Bevorzugt handelt es sich bei R⁷ um Gruppen R⁷ mit R¹⁰ = H, d.h. vom Ethylenoxid abgeleiteten Gruppen.

In einer weiteren bevorzugten Ausführungsform der Erfindung handelt es sich bei R² um Reste der allgemeinen Formel -(-CH₂CH(CH₂OH)-O-)_{c}(CH₂CH(R¹⁴)-O-)_{d}-R⁶-X (IIIe), wobei die Summe aus c+d = z. In der Formel (IIIe) handelt es sich um Monomere R⁷ und R⁸, wobei R¹¹ für H steht. Bei den Resten R¹⁴ in der Formel (IIIe) handelt es sich unabhängig voneinander um einen Rest ausgewählt aus der Gruppe von H, Methyl oder Ethyl, wobei es sich bei mindestens 20 % der Reste R¹⁰ um H handelt, bevorzugt bei mindestens 50 % der Reste, besonders bevorzugt bei mindestens 80 % der Reste und besonders bevorzugt handelt es sich bei allen Resten R¹⁴ um H. Bei R⁶ handelt es sich bei dieser Ausführungsform bevorzugt um eine Einfachbindung und bei X bevorzugt um H.
In einer weiteren Ausführungsform der Erfindung handelt es sich um Derivate von Tris(2-hydroxyphenyl)methanen, welche erhältlich sind, indem man Carbonsäurevinylester in Gegenwart von Verbindungen der allgemeinen Formeln (I) oder (II) radikalisch polymerisiert.

Die Polymerisation von Carbonsäurevinylestern in Gegenwart Polyalkoxygruppen ist dem Fachmann prinzipiell bekannt. Bei dieser Reaktion pfropfen Vinylester-, Oligovinylester- und/oder Polyvinylestergruppen auf die Polyalkoxygruppen auf, d.h. es entstehen zusätzliche Seitengruppen aufweisende Polyalkoxygruppen. Die Polyvinylestergruppen werden anschließend zumindest teilweise zu OH-Gruppen hydrolysiert. Die OH-Gruppen können anschließend optional mit Gruppen -R⁶-X funktionalisiert werden. Das Reaktionsschema ist nachfolgend beispielhaft illustriert.

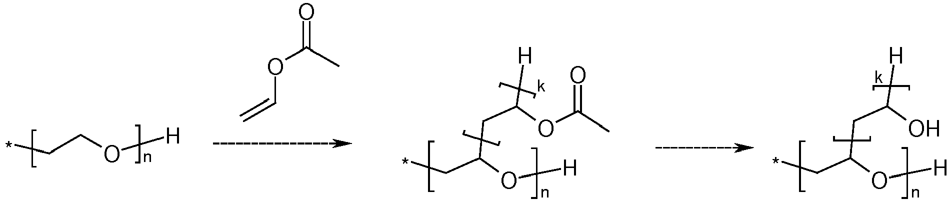

Geeignete Carbonsäurevinylester umfassen insbesondere Vinylformiat, Vinylacetat und Vinylpropionat. Zur Modifizierung besonders geeignet sind insbesondere Verbindungen, welche Gruppen (IIIb) oder (IIIc) aufweisen.

Zur Herstellung der erfindungsgemäßen Verbindungen können zunächst Tris(2-hydroxyphenyl)methan-Verbindungen der allgemeinen Formeln (IV) oder (V) mit dem gewünschten Substitutionsmuster hinsichtlich R¹, und R bzw. R³ und R⁴ synthetisiert werden.

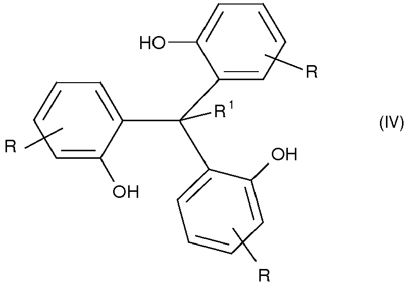

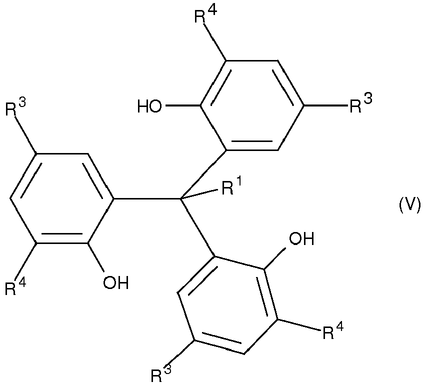

Die Methoden zu Herstellung der Verbindungen sind in der eingangs zitierten Literatur, beispielsweise G. Casiraghi (Tetrahedron Letters, No. 9, 679 bis 682 (1973)), M. B. Dinger (Chem. Commun., 1999, 2525/2526, Inorg. Chem. 2000, 39, 1238 bis 1254) sowie (Inorg. Chem. 2001, 40, 1029 bis 1036), M. B. Dinger (Eur J. Org. Chem. 2000, 2467 bis 2478), K. Matloka (Dalton Trans., 2005, 3719 bis 3721), M. W. Peters (Inorg. Chem., 2002, 41, 1701 bis 1716) und von R. Mitra (Dalton Trans., 2007, 3924 bis 3935) ausführlich beschrieben.

Die Tris(2-hydroxyphenyl)methan-Verbindungen der allgemeinen Formeln (IV) oder (V) können in einem zweiten Schritt in prinzipiell bekannter Art und Weise alkoxyliert werden. Die Durchführung von Alkoxylierungen ist dem Fachmann prinzipiell bekannt. Es ist dem Fachmann ebenfalls bekannt, dass man durch die Reaktionsbedingungen, insbesondere die Wahl des Katalysators, die Molekulargewichtsverteilung der Alkoxylate beeinflussen kann. Zur Alkoxylierung werden C₂- bis C₈-Alkylenoxide eingesetzt, beispielsweise, Ethylenoxid, Propylenoxid, Butylenoxid oder Styroloxid. Bevorzugt sind jeweils die 1,2-Alkylenoxide. Die Verwendung dieser Alkylenoxide führt zu Gruppen, welche Reste R⁷ umfassen.

Um Reste R⁸ und R⁹ zu erhalten, setzt man Glycidol (VIa) oder mit einer geeigneten Schutzgruppe R¹⁰ geschütztes Glycidol (Vlb) 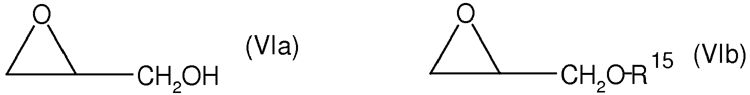 als Alkenoxid ein. Bei R¹⁰ kann es sich prinzipiell um alle Arten von Gruppen handeln, mit denen sich die OH-Funktion während der Alkoxylierung schützen lässt. Sie können nach der Alkoxylierung oder auch erst nach Einführung der Gruppen -R⁶-X in prinzipiell bekannter Art und Weise abgespalten werden. Bei R¹⁵ kann es sich beispielsweise um eine t-Butylgruppe oder eine Benzylgruppe handeln.

Da nach der Reaktion des ungeschützten Glycidols zwei OH-Gruppen in der Kette vorhanden sind, welche weiterreagieren können, entstehen die bereits zitierten Verzweigungen im Rest R². Mithilfe des geschützten Glycidols lassen sich gezielt Gruppen R⁸ der Formel - CH₂-CH(CH₂OH)- in den Rest R² einführen.

Bei der Alkoxylierung kann es sich um eine basenkatalysierte Alkoxylierung handeln. Dazu können die Tris(2-hydroxyphenyl)methan-Verbindungen in einem Druckreaktor mit Alkalimetallhydroxiden, bevorzugt Kaliumhydroxid oder mit Alkalialkoholaten wie beispielsweise Natriummethylat versetzt werden. Durch verminderten Druck (bspw. <100 mbar) und/oder Erhöhung der Temperatur (30 bis 150°C) kann noch in der Mischung vorhandenes Wasser abgezogen werden. Der Alkohol liegt danach als das entsprechende Alkoholat vor. Anschließend wird mit Inertgas (z.B. Stickstoff) inertisiert und das oder die Alkylenoxid(e) bei Temperaturen von 60 bis 180°C bis zu einem Druck von max. 10 bar schrittweise zugegeben. Am Ende der Reaktion kann der Katalysator durch Zugabe von Säure (z.B. Essigsäure oder Phosphorsäure) neutralisiert und kann bei Bedarf abfiltriert werden.

Der Katalysator kann auch durch Zugabe handelsüblicher Silicate, welchen anschließend abfiltriert werden, entfernt werden. Optional kann die Alkoxylierung auch in Gegenwart eines Lösungsmittels durchgeführt werden. Dies kann z.B. Toluol, Xylol, Dimethylformamid oder Ethylencarbonat sein. Die Alkoxylierung kann auch durch Zugabe von Kronenethern, beispielsweise durch die Zugabe von [18]-Krone-6, beschleunigt werden.

Die Alkoxylierung der Alkohole kann aber auch mittels anderer Methoden vorgenommen werden, beispielsweise durch säurekatalysierte Alkoxylierung. Die Säuren können Protonensäuren oder Lewis-Säuren (wie beispielsweise BF₃ sein. Weiterhin können beispielsweise Doppelhydroxid-Tone wie in DE 43 25 237 A1 beschrieben eingesetzt werden, oder es können Doppelmetallcyanid-Katalysatoren (DMC-Katalysatoren) verwendet werden.

Geeignete DMC-Katalysatoren sind beispielsweise in der DE-A 102 43 361, insbesondere den Abschnitten [0029] bis [0041] sowie der darin zitierten Literatur offenbart. Beispielsweise können Katalysatoren vom Zn-Co-Typ eingesetzt werden. Zur Durchführung der Reaktion kann der Alkohol R-OH mit dem Katalysator versetzt, die Mischung wie oben beschrieben entwässert und mit den Alkylenoxiden wie beschrieben umgesetzt werden. Es werden üblicherweise nicht mehr als 1000 ppm Katalysator bezüglich der Mischung eingesetzt, und der Katalysator kann aufgrund dieser geringen Menge im Produkt verbleiben.

Die Katalysatormenge kann in der Regel geringer sein als 1000 ppm, beispielsweise 250 ppm und weniger.

Die Alkoxylierung kann alternativ auch durch Reaktion der Verbindungen (IV) und (V) mit cyclischen Carbonaten wie beispielsweise Ethylencarbonat vorgenommen werden.

Mittels der Alkoxylierung erhält man bereits erfindungsgemäße Verbindungen, nämlich solche mit X = H. Diese weisen terminale OH-Gruppen auf. Dies ist in den nachfolgenden Abbildungen (VII) bzw. (VIII) beispielhaft anhand von erfindungsgemäßen Verbindungen, welche Polyalkoxyketten aus Gruppen R⁷ aufweisen gezeigt.

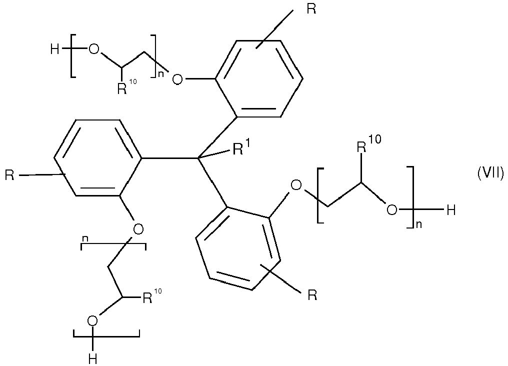

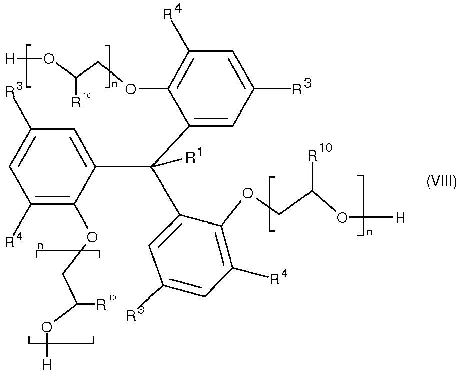

Zur Einführung von Gruppen X, welche nicht H sind, werden die alkoxylierten, terminale OH-Gruppen aufweisenden Tris(2-hydroxyphenyl)methan-Derivate der Formel (VII) bzw. (VIII) auf geeignete Art und Weise weiter mit Gruppen -R⁶-X funktionalisiert. Hierdurch werden Verbindungen der allgemeinen Formel (IX) bzw. (X) erhalten.

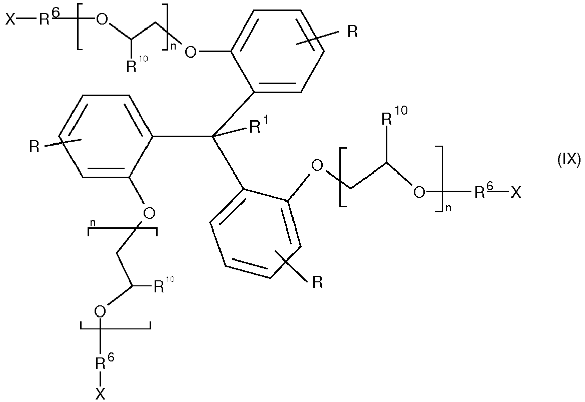

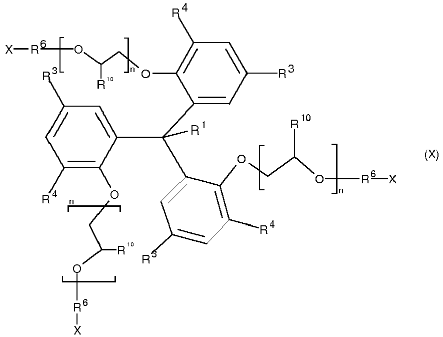

Sulfatgruppen -OSO₃M umfassende Derivate können erhalten werden durch Umsetzung der terminalen OH-Gruppen mit SO₃, Schwefelsäure, Chlorschwefelsäure oder Aminosulfonsäure (CAS-Nr. 5329-14-6) und nachfolgender Neutralisation mit z.B. Natronlauge. Dies kann z.B. in einem Fallfilmreaktor durchgeführt werden. Durch diese Reaktion werden nur die terminalen OH-Gruppen durch Sulfatgruppen substituiert. R⁶ steht bei dieser Reaktion für eine Doppelbindung.

Sulfonatgruppen -SO₃M umfassende Derivate können erhalten werden durch Substitution der OH-Gruppe gegen Cl unter Verwendung von Phosgen oder Thionylchlorid. Die Umsetzung kann in Gegenwart eines Lösungsmittels wie z.B. Chlorbenzol vorgenommen werden. Dabei frei werdende HCl sowie freiwerdendes CO₂ oder SO₂ kann vorteilhaft durch Strippen mit Stickstoff aus dem System entfernt werden, so dass eine Etherspaltung unterbunden wird. Die Alkylalkoxychlor-Verbindung wird im Anschluss daran mit einer wässrigen Lösung von Natriumsulfit umgesetzt, wobei das Chlorid durch Sulfit substituiert wird und das Sulfonat erhalten wird. Die Substitution kann in Gegenwart eines Phasenvermittlers (bspw. C₁- bis C₈-Alkohole) bei einer Temperatur 100 bis 180°C und Druck vorgenommen werden.

Die Sulfonate können alternativ durch Addition von Vinylsulfonsäure an die Verbindung (V) (oder auch an VII bzw. VIII) erhalten werden. Einzelheiten hierzu sind beispielsweise in EP-A 311 961 beschrieben. Sulfonate können weiterhin erhalten werden, indem man die Verbindungen (V) (oder auch an VII bzw. VIII) mit 1,3-Propansulton oder 1,4-Butansulton umsetzt. Hierbei werden Sulfonate mit einer terminalen Gruppe -CH₂-CH₂-CH₂-SO₃M (d.h. R⁶ = CH₂-CH₂-CH₂-) bzw. -CH₂ -CH₂-CH₂-CH₂-SO₃M (d.h. R⁶ = CH₂-CH₂-CH₂-CH₂-) erhalten. Verbindungen mit einer terminalen Gruppe -CH₂-CH(OH)-CH₂-SO₃M (d.h. R⁶ = - CH₂-CH(OH)-CH₂-) lassen sich durch Umsetzung der Verbindung (V) mit Epichlorhydrin und nachfolgender nucleophiler Substitution der Chloridgruppe durch Natriumsulfit erhalten.

Carboxylatgruppen -COOM umfassende Derivate können durch Oxidation der Verbindung (V) (oder auch an VII bzw. VIII) erhalten werden. Hierzu sind alle Oxidationsmittel geeignet, gegebenenfalls in Verbindung mit geeigneten Katalysatoren, welche die terminale OH-Gruppe der Verbindung (V) zur COOH-Gruppe oxidieren können, ohne in großem Maße andere Teile des Moleküls zu oxidieren. Die Oxidation kann beispielsweise mit Hilfe von Luft oder Sauerstoff unter Verwendung eines Edelmetallkatalysators (beispielsweise eines Katalysators auf Basis von Palladium) vorgenommen werden. Bei dieser Synthesevariante wird eine terminale Gruppe -CH₂-COOM erhalten (d.h. R⁶ = -CH₂-). Carboxylate können weiterhin auch hergestellt werden, indem man an die OH-Gruppen mittels einer Michael-Addition (Meth)acrylsäure oder einen (Meth)acrylsäureester addiert. Falls die Ester verwendet werden, werden diese nach der Addition verseift. Bei dieser Synthesevariante werden - je nachdem, ob Acrylsäure oder (Meth)acrylsäure bzw. deren Ester verwendet wurden - terminale Gruppen -CH₂-CH₂-COOM oder -CH₂-CH(CH₃)-COOM erhalten. Carboxalatgruppen COOM könnnen auch durch Umsetzung der Verbindungen (VII) oder (VIII) mit Chloressigsäure ClCH₂COOHoder deren Salzen ClCH₂COOM in Gegenwart einer Base erhalten werden.

Phosphatgruppen lassen sich durch Umsetzung mit Phosphorpentoxid einführen, Phosphonatgruppen durch Umsetzung mit Vinylphosphonsäure.

Verbindungen mit Mono- oder Oligosaccharidgruppen können hergestellt werden, indem man das entsprechend Saccharid, beispielsweise Glucose mit Hilfe eines sauren Katalysators, wie beispielsweise para-Toluolsulfonsäure und n-Butanol in das entsprechende Butylacetal überführt. Das entstehende Reaktionswasser kann durch Anlegen von Vakuum aus dem Reaktionsgemisch entfernt werden. Im Anschluss wird die Verbindung (V) (oder auch an VII bzw. VIII) zugegeben und die Umacetalisierung durch destillative Entfernung des Butanols aus dem Gleichgewicht vorangetrieben. Der saure Katalysator kann am Ende der Reaktion durch Zugabe von Base, beispielsweise NaOH oder KOH neutralisiert werden.

Je nach Art der Gruppen R² weisen die erhaltenden Verbindungen nur eine terminale Gruppe -R⁶-X auf oder auch mehrere terminale und/oder seitenständige Gruppen -R⁶-X.

Bei der Einführung der terminalen Gruppe -R⁶-X müssen selbstverständlich nicht alle OH-Gruppen der H-terminierten erfindungsgemäßen Verbindungen umgesetzt werden. Es ist möglich, nur einen Teil der Gruppen, beispielsweise nur durchschnittlich jede dritte Gruppe umzusetzen. Auf diese Art und Weise lassen sich die Eigenschaften der erfindungsgemäßen Verbindungen an den gewünschten Verwendungszweck anpassen.

Bei Verwendung von Glycidol sind verschiedene Synthesevarianten denkbar. Verwendet man ungeschütztes Glycidol, so können die Gruppen R² verzweigt sein und mehrere terminale oder seitenständige OH-Gruppen aufweisen. Diese Gruppen können ganz oder auch nur teilweise zur Gruppen -R⁶-X umgesetzt werden. Bei einer nur teilweisen Umsetzung erfolgt die Umsetzung statistisch.

Verwendet man geschütztes Glycidol (Vlb) so entsteht zunächst einmal eine unverzweigte Polyalkoxykette mit einer terminalen OH-Gruppe und seitenständigen, geschützten OH-Gruppen. Man kann nun einerseits die Schutzgruppen zunächst abspalten und dann die Einführung der Gruppen -R⁶-X vornehmen.
In diesem Falle entsteht eine lineare Gruppe R², welche end- und/oder seitenständig Gruppen -R⁶-X aufweist. Spaltet man in einer alternativen Synthese die Schutzgruppen zunächst nicht ab, sondern nimmt erst die Einführung der Gruppen -R⁶-X vor, dann reagieren nur die terminalen OH-Gruppen. Die Abspaltung der Schutzgruppen kann danach erfolgen. In diesem Falle entsteht eine Gruppe R², welche eine terminale Gruppe -R⁶-X aufweist und darüber hinaus seitenständige Methylolgruppen -CH₂OH.

Zur Ausführung des erfindungsgemäßen Verfahrens werden in die Erdöllagerstätte mindestens eine Produktionsbohrung und mindestens eine Injektionsbohrung abgeteuft. In der Regel wird eine Lagerstätte mit mehreren Injektionsbohrungen und mit mehreren Produktionsbohrungen versehen.

Durch die mindestens eine Injektionsbohrung wird eine wässrige Formulierung des beschriebenen Tris(2-hydroxyphenyl)-methan-Derivats (I) in die Erdöllagerstätte injiziert und der Lagerstätte durch mindestens eine Produktionsbohrung Erdöl entnommen. Mit dem Begriff "Erdöl" ist in diesem Zusammenhang nicht nur phasenreines Öl gemeint, sondern der Begriff umfasst auch die üblichen Rohöl-Wasser-Emulsionen. Durch den durch die eingepresste Formulierung erzeugten Druck, fließt das Erdöl in Richtung der Produktionsbohrung und wird über die Produktionsbohrung gefördert.

Die Lagerstätten-Temperatur der Erdöllagerstätte, auf die das erfindungsgemäße Verfahren angewandt wird, beträgt erfindungsgemäß 10 bis 150°C, bevorzugt 10°C bis 120°C und beispielsweise 20°C bis 70°C.

Der Fachmann weiß, dass eine Erdöllagerstätte oftmals, außer bei Thermal-Maßnahmen, eine zeitlich und Areal-bezogen gleichmäßige Temperatur aufweist. Die genannte Lagerstätten-Temperatur bezieht sich auf den Bereich der Lagerstätte zwischen den Injektions- und Produktionsbohrungen, der von der injizierten Zusammensetzung erfasst wird. Methoden zur Ermittlung der Temperatur einer Erdöllagerstätte sind dem Fachmann prinzipiell bekannt. Die Temperatur wird in der Regel aus Temperaturmessungen an bestimmten Stellen der Formation vorgenommen.

Das erfindungsgemäße Verfahren kann insbesondere bei Erdöllagerstätten mit einer durchschnittlichen Permeabilität von 100 mD bis 154 D, bevorzugt 150 mD bis 2 D und besonders bevorzugt 200 mD bis 1 D angewandt werden. Die Permeabilität einer Erdölformation wird vom Fachmann in der Einheit "Darcy" (abgekürzt "D" bzw. "mD" für "Millidarcy") angegeben und kann aus der Fließgeschwindigkeit einer flüssigen Phase in der Erdölformation in Abhängigkeit der angelegten Druckdifferenz bestimmt werden. Die Fließgeschwindigkeit kann in Kernflutversuchen mit der Formation entnommenen Bohrkernen bestimmt werden.
Einzelheiten hierzu finden sich beispielsweise in K. Weggen, G. Pusch, H. Rischmüller in "Oil and Gas", Seiten 37 ff., Ulmann's Encyclopedia of Industrial Chemistry, Online-Ausgabe, Wiley-VCH, Weinheim 2010. Für den Fachmann ist klar, dass die Permeabilität in einer Erdöllagerstätte nicht homogen sein muss, sondern im Allgemeinen eine gewisse Verteilung aufweist, und es sich dementsprechend bei der Angabe der Permeabilität einer Erdöllagerstätte um eine durchschnittliche Permeabilität handelt.

Zur Ausführung des Verfahrens wird eine wässrige Formulierung eingesetzt, welche neben Wasser mindestens das beschriebene Tris(2-hydroxyphenyl)-methan-Derivat (I) umfasst. Es können auch Gemische verschiedener Tris(2-hydroxyphenyl)-methan-Derivats eingesetzt werden. Die Formulierung kann in Süßwasser aber auch in Salzen enthaltendem Wasser angesetzt werden. Es kann es sich um Mischungen verschiedener Salze handeln.

Beispielsweise kann Meerwasser zum Ansetzen der wässrigen Formulierung verwendet werden oder es kann gefördertes Formationswasser verwendet werden, welches auf diese Art und Weise wieder verwendet wird. Bei Förderplattformen im Meer wird die Formulierung in der Regel in Meerwasser angesetzt. Bei Fördereinrichtungen an Land kann das Tris(2-hydroxyphenyl)-methan-Derivat vorteilhaft zunächst in Süßwasser gelöst und die erhaltene Lösung mit Formationswasser auf die gewünschte Einsatzkonzentration verdünnt werden. Die Formulierung kann bevorzugt hergestellt werden, indem man das Wasser vorlegt, das Tris(2-hydroxyphenyl)-methan-Derivat als Pulver einstreut und mit dem Wasser vermischt.

Bei den Salzen kann es sich insbesondere um Alkalimetallsalze sowie Erdalkalimetallsalze handeln. Beispiele typischer Kationen umfassen Na⁺, K⁺, Mg²⁺ oder Ca²⁺ und Mg²⁺. Beispiele typischer Anionen umfassen Chlorid, Bromid, Hydrogencarbonat, Sulfat oder Borat.

Sofern die Formulierung Salze umfasst, sind in der Regel zumindest eines oder mehrere Alkalimetallionen, insbesondere zumindest Na⁺ vorhanden. Daneben können auch noch Erdalkalimetallionen vorhanden sein, wobei das Gewichtsverhältnis Alkalimetallionen / Erdalkalimetallionen in der Regel ≥ 2, bevorzugt ≥ 3 ist. Als Anionen sind in der Regel zumindest eines oder mehrere Halogenid-Ionen, insbesondere zumindest Cl⁻ vorhanden. In der Regel beträgt die Menge an Cl⁻ zumindest 50 Gew. %, bevorzugt mindestens 80 Gew. % bezüglich der Summe aller Anionen.

Die Gesamtmenge aller Salze in der wässrigen Formulierung beträgt häufig 10 000 ppm bis 350 000 ppm (Gewichtsanteile), bezüglich der Summe aller Komponenten der Formulierung.

Sofern Meerwasser zum Ansetzen der Formulierung verwendet wird, beträgt der Salzgehalt in der Regel 20 000 ppm bis 50 000 ppm und sofern Formationswasser verwendet wird in der Regel 100 000 ppm bis 250 000 ppm. Die Menge an Erdalkalimetallionen kann bevorzugt 1000 bis 53 000 ppm betragen. Die wässrige Formulierung kann auch weitere Komponenten umfassen, wie z. B. Biozide, Stabilisatoren und Inhibitoren.

Die Konzentration des Tris(2-hydroxyphenyl)-methan-Derivats wird so festgelegt, dass die wässrige Formulierung die gewünschte Viskosität für den Einsatzzweck aufweist. Die Viskosität der Formulierung sollte in der Regel mindestens 3 mPas (gemessen bei 25°C und einer Scherrate von beispielsweise 7 s⁻¹, beziehungsweise wie sie im Reservoir bevorzugt auftritt) betragen, bevorzugt mindestens 10 mPas.

Erfindungsgemäß beträgt die Konzentration des Tris(2-hydroxyphenyl)-methan-Derivats in der Formulierung 0,01 bis 10 Gew.-%, oftmals 0,05 bis 10 Gew.-% bezüglich der Summe aller Komponenten der wässrigen Formulierung. Bevorzugt beträgt die Menge 0,05 bis 5 Gew. %, besonders bevorzugt 0,05 bis 1 Gew. % und beispielsweise ca. 0,1 Gew. %.

Das Injizieren der wässrigen Formulierung kann mittels üblicher Vorrichtungen vorgenommen werden.

Die Formulierung kann mittels üblicher Pumpen in eine oder mehrere Injektionsbohrungen injiziert werden. Die Injektionsbohrungen sind oftmals im Bereich der Erdöllagerstätte mit einzementierten Stahlrohren ausgekleidet, und die Stahlrohre sind an der gewünschten Stelle perforiert. Die Formulierung tritt durch die Perforation aus der Injektionsbohrung in die Erdölformation ein. Über den mittels der Pumpen angelegten Druck wird in prinzipiell bekannter Art und Weise die Strömungsgeschwindigkeit der Formulierung und damit auch die Scherbelastung festgelegt, mit der die wässrige Formulierung in die Formation eintritt. Die Scherbelastung beim Eintritt in die Formation kann vom Fachmann in prinzipiell bekannter Art und Weise auf Basis des Gesetz' von Hagen-Poiseuille unter Verwendung der beim Eintritt in die Formation durchströmten Fläche, dem mittleren Porenradius und dem Volumenstrom errechnet werden. Die durchschnittliche Permeabilität bzw. Porosität der Formation kann in prinzipiell bekannter Art und Weise durch Messungen an Bohrkernen ermittelt werden. Die Scherbelastung ist naturgemäß umso größer, je größer der in die Formation injizierte Volumenstrom an wässriger Formulierung ist.

Die Geschwindigkeit der Injektion kann vom Fachmann je nach den Eigenschaften der Formation (Permeabilität, Mächtigkeit) sowie den Erfordernissen des Erdölfeldes (Anzahl der Injektoren, deren Konfiguration, etc.) festgelegt werden.
Bevorzugt beträgt die Scherrate beim Eintritt der wässrigen Formulierung in die Formation mindestens 30000 s⁻¹, bevorzugt mindestens 60000 s⁻¹ und besonders bevorzugt mindestens 90000 s⁻¹.

Zur Ausführung des Verfahrens besonders bevorzugte Tris(2-hydroxyphenyl)-methan-Derivate der Formel (I) sind die nachfolgenden Komponenten.

Diese Produkte können als Feststoffe gelagert und transportiert werden, sie sind per se lagerstabil, können aber auch als wässrige Lösungen oder Zusammensetzungen mit weiteren Komponenten bereitgestellt werden.

In den nachfolgend genannten Produkten wird das Grundelement Tris-(3,5-di-*tert-*butyl-2-hydroxyphenyl)-methan mit TRIS abgekürzt wird:
TRIS[(-CH₂-CH₂-O)₁₁H]₃
TRIS[(-CH₂-CH₂-O)₁₁H]₂[(-CH₂-CH₂-O)₁₁-SO₃Na]
TRIS[(-CH₂-CH₂-O)₁₁-SO₃Y]₃ (Y = Na⁺, NH₄⁺)
TRIS[(CH₂CH(CH₃)-O)_{4,4}-(-CH₂-CH₂-O)₁₃H]₃
TRIS mit 24 Glycidol-Einheiten und 9 EO-Einheiten
TRIS[(CH₂CH₂-O)₈-(-CH₂-CH(CH₂OH)-O)₃H]₃
TRIS[(CH₂CH₂-O)₈-(-CH₂-CH(CH₂OH)-O)₂H]₃
TRIS[(CH₂CH₂-O)₁₁-(-CH₂-CH(CH₂OH)-O)₁H]₃
TRIS[(CH₂CH₂-O)₇-(-CH₂-CH(CH₂OH)-O)₄H]₃
TRIS[(CH₂CH₂-O)₆-(-CH₂-CH(CH₂OH)-O)₄H]₃
TRIS[(CH₂CH₂-O)₅-(-CH₂-CH(CH₂OH)-O)₅H]₃
TRIS[(CH₂CH₂-O)₁₀H]₃
TRIS[(CH₂CH₂-O)₉-(-CH₂COONa)]₃

Die Erfindung betrifft auch eine wässrige Formulierung enthaltend 0,1 bis 90 Gew. % eines Tris(2-hydroxyphenyl)methan-Derivats aus der o.g. genannten Gruppe, sowie enthaltend 50 000 ppm bis 350 000 ppm anorganisches Salz, sowie (ggf.) 0,01 bis 5 Gew. % mindestens einer Hilfskomponente aus der Gruppe Tenside, Antioxidantien und Biozide, sowie Wasser und ggf. weitere Lösungsmittel.

Die beschriebenen Derivate (I) von Tris-(2-hydroxyphenyl)-methan können zur Herstellung verschiedener Formulierungen für die Erdölförderung wie oben beschrieben verwendet werden. Besonders geeignet sind auch die TRIS-Derivate, die sowohl EO-Einheiten wie auch Glycidol-Einheiten enthalten.

Häufig eingesetzte Konzentrationen und weitere Komponenten:
A) Konzentrationen liegen oft im Bereich von 0,01-10 Gew.-%, oftmals 0,05-10 Gew.-%, bevorzugte Konzentrationen sind zwischen 0,1 - 1 Gew.-% und besonders bevorzugte Konzentrationen zwischen 0,1 und 0,5 Gew.-%, jeweils bezogen auf die gesamte Formulierung.
B) Lösemittel können als weitere Komponente eingesetzt werden. Typischerweise wird das Derivat von Tris-(2-hydroxyphenyl)-methan im Formationswasser gelöst. Auch ein Lösen in Meerwasser ist möglich. Ein Vor-Lösen mit einem Wasser-mischbaren Lösungsmittel, z. B. Ethanol oder Isopropanol zur Herstellung von Konzentraten mit höherem Aktivgehalt ist möglich. Auch Brunnenwasser kann verwendet werden.
C) Salze haben einen Einfluss auf die Viskosität der Formulierungen. Die Ziel-Viskosität wird oft unter Lagerstätten-Salinität über Variation der Konzentration eingestellt.
D) Eine Abhängigkeit vom pH-Wert der Formulierung auf die verdickenden Eigenschaften bzw. die Viskosität (z. B. bei Einsatz von Carboxylaten) ist möglich.
E) Eine Kombination des Derivats von Tris-(2-hydroxyphenyl)-methan mit einem oder mehreren weiteren Tensid ist möglich.
F) Optional können auch weitere Komponenten, wie Biozide in der Formulierung eingesetzt werden. In der Regel werden Biozide schon für das Watertreatment eingesetzt. Insbesondere niedrig-salinare Wässer können mit Algiziden, Fungiziden, etc. behandelt werden.

Die folgenden Beispiele und die Ansprüche sollen die Erfindung näher erläutern.

### Beispiel 1 Synthese von Tris(2-hydroxyphenyl)-methan-Derivaten

### 1.1 Synthese von Tris-(3,5-di-tert-butyl-2-hydroxyphenyl)-methan

Tris-(3,5-di-*tert-*butyl-2-hydroxyphenyl)-methan (CAS-No.143560-44-5) wurde mittels des von M. B. Dinger, M. J. Scott, Eur. J. Org. Chem. 2000, 2467 beschriebenen Verfahrens hergestellt. Tris-(3,5-di-*tert-*butyl-2-hydroxyphenyl)-methan wird nachfolgend auch als TRIS abgekürzt.

### 1.2 Synthese von TRIS[(-CH₂-CH₂-O)₁₁H]₃ durch Ethoxylierung von TRIS mit 33 EO-Einheiten

In einem 2l Autoklaven wird TRIS (100g) in Toluol (150 ml) suspendiert mit Kalium-tert-butylat (3.0 g) versetzt. Es wird dreimal mit N₂ gespült, ein Vordruck von ca. 1.3 bar N₂ eingestellt und die Temperatur auf 119-121 °C erhöht. Ethylenoxid (231 g) wird so zudosiert, dass die Temperatur zwischen 119°C-130°C bleibt. Anschließend wird 16 h bei 90°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Das Lösungsmittel wird mit Hilfe eines Rotationsverfahrens entfernt und das Produkt wird anschließend für 5 Stunden bei 80°C und einem Druck p=20 mbar nachgetrocknet.

Man erhält 263 g Zwischenprodukt, das eine OH-Zahl von 105,9 mg/g aufweist. In einem zweiten Schritt werden 239 g des so erhaltenen Zwischenproduktes in einem 2l Autoklaven mit Kalium-tert-butylat (1,5 g) versetzt. Es wird dreimal mit N₂ gespült, ein Vordruck von ca. 1.3 bar N₂ eingestellt und die Temperatur auf 119-121 °C erhöht. Ethylenoxid (74 g) wird so zudosiert, dass die Temperatur zwischen 119°C-130°C bleibt. Anschließend wird 16 h bei 100°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Auf diese Weise erhält man 307 g der Zielverbindung. OH-Zahl: 87,5 mg/g. Das TAI-¹H-NMR (siehe J. Loccufier, M. van Bos, E. Schacht, Polymer Bulletin, 1991, 27, 201) der Substanz zeigt einen mittleren Ethoxylierungsgrad von 11 Ethylenoxid-Einheiten pro phenolischer OH-Gruppe.
Alternativ kann das Produkt auch in Gegenwart von Kronenether [18]Krone-6 wie folgt hergestellt werden:

In einem 2 I Autoklaven wird TRIS (80 g) in Toluol (220 ml) suspendiert und mit Kalium-tert-butylat (2,2 g) sowie Kronenether [18]Krone-6 (5,3 g) versetzt.
Es wird dreimal mit N₂ gespült, ein Vordruck von ca. 1,3 bar N₂ eingestellt und die Temperatur auf 130°C erhöht. Ethylenoxid (185 g) wird innerhalb von 3 h zudosiert, wobei die Temperatur bei 130°C gehalten wird. Anschließend wird 8 h bei 130°C und dann über Nacht bei 50°C nachgerührt. Flüchtige organische Verbindungen werden durch einen Stickstoffstrom entfernt und der Reaktor wird entleert. Das basische Rohprodukt wird mit Hilfe von handelsüblichen Mg-Silikaten neutralisiert, welche anschließend abfiltriert werden. Das Lösungsmittel wird mit Hilfe eines Rotationsverdampfers entfernt und das Produkt wird anschließend für 5 h bei 80°C und p = 20 mbar nachgetrocknet. Man erhält 277 g des Produktes. SEC zeigt Mₙ = 2080 g/mol und M_{w} = 2136 g/mol und eine Polydispersität M_{w}/Mₙ = 1.03. Das Produkt wird auch mittels ¹H-NMR charakterisiert.

### 1.3 Synthese von TRIS[(-CH₂-CH₂-O)₁₁H]₂[(-CH₂-CH₂-O)₁₁-SO₃Na] durch Sulfatierung

Das in Beispiel 1.2 synthetisierte Produkt TRIS[(-CH₂-CH₂-O)₁₁H]₃ wird in Dichlormethan (45 g) gelöst und auf 5-10°C abgekühlt. Danach wird Chlorsulfonsäure (2,12 g) so zugetropft, dass die Temperatur 10°C nicht übersteigt. Man lässt 1 h bei 10°C, danach über Nacht bei Raumtemperatur rühren, bevor obiges Reaktionsgemisch in eine wässrige NaOH-Lösung (50%, 1,98 g) bei max. 10°C getropft wird. Das Dichlormethan wird unter leichtem Vakuum am Rotationsverdampfer bei 30-45°C entfernt. Das Produkt wird per ¹H-NMR charakterisiert und der Wassergehalt der Lösung bestimmt (ca. 60%).

### 1.4 Synthese von TRIS[(-CH₂-CH₂-O)₁₁-SO₃Y]₃ (Y = Na⁺, NH₄⁺) durch Ethoxylierung von TRIS mit 33 EO-Einheiten und anschließender vollständiger Sulfatierung

Das Produkt aus Synthesebeispiel 1.2 (30 g), Aminosulfonsäure (CAS 5329-14-6; 5.0 g) und katalytische Mengen Harnstoff (17 mg) werden für 7 h auf 85°C erhitzt. Nach Abkühlen auf Raumtemperatur werden 35 g Wasser zugegeben und der pH-Wert der so erhaltenen Lösung wird mit 50%-iger Natronlauge auf pH = 9 eingestellt. Das Produkt wird mittels ¹H-NMR charakterisiert und der Wassergehalt der Lösung bestimmt (ca. 47%).

### 1.5 Synthese von TRIS[(CH₂CH(CH₃)-O)_{4,4}-(-CH₂-CH₂-O)₁₃H]₃ durch Propoxylierung von TRIS mit 13,2 PO-Einheiten und anschließender Ethoxylierung mit 39 EO Einheiten

In einem 2 I Autoklaven wird TRIS (50 g) in Toluol (130 ml) suspendiert und mit Kalium-tert-butylat (1,4 g) sowie Kronenether [18]Krone-6 (3,3 g) versetzt.
Es wird dreimal mit N₂ gespült, ein Vordruck von ca. 1,3 bar N₂ eingestellt und die Temperatur auf 130°C erhöht. Propylenoxid (61 g) wird innerhalb von 80 min. zudosiert, wobei die Temperatur bei 130°C gehalten wird. Anschließend wird 8 h bei 130°C und dann über Nacht bei 50°C nachgerührt. Am nächsten Tag wird das Reaktionsgemisch auf 130°C erhitzt und Ethylenoxid (136,4 g) wird über einen Zeitraum von 2 h zudosiert, wobei die Temperatur bei 130°C gehalten wird. Anschließend wird 8 h bei 130°C und dann über Nacht bei 50°C nachgerührt.

Flüchtige organische Verbindungen werden durch einen Stickstoffstrom entfernt und der Reaktor wird entleert. Das basische Rohprodukt wird mit Hilfe von handelsüblichen Mg-Silikaten neutralisiert, welche anschließend abfiltriert werden. Das Lösungsmittel wird mit Hilfe eines Rotationsverdampfers entfernt und das Produkt wird anschließend für 2 h bei 80°C und p = 20 mbar nachgetrocknet. Man erhält 236 g des Produktes. SEC zeigt Mₙ = 2965 g/mol und M_{w} = 3139 g/mol und eine Polydispersität M_{w}/Mₙ = 1.1. Das Produkt wird auch mittels ¹H-NMR charakterisiert.

### 1.6 Alkoxylierung von TRIS mit 24 Glycidol-Einheiten und 9 Ethylenoxid-Einheiten

In einem Autoklaven wird TRIS (50 g) in Toluol (100 g) suspendiert und mit Kalium-*tert-*butylat (1.7 g) sowie Kronenether [18]Krone-6 (4 g) versetzt. Es wird dreimal mit N₂ gespült, ein Vordruck von ca. 1.3 bar N₂ eingestellt und die Temperatur auf 120°C erhöht. Eine Mischung von Glycidol (142 g) und Ethylenoxid (31.5 g) wird über einen Zeitraum von 8 h so zudosiert, dass die Temperatur bei 120°C bleibt. Anschließend wird 16 h bei 80°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Das Lösungsmittel wird mit Hilfe eines Rotationsverfahrens entfernt und das Produkt wird anschließend für 5 Stunden bei 80°C und einem Druck p=20 mbar nachgetrocknet. Man erhält 200 g des Zielproduktes. Das Produkt wird mittels ¹H-NMR charakterisiert.

### 1.7 Synthese von TRIS[(CH₂CH₂-O)₈-(-CH₂-CH(CH₂OH)-O)₃H]₃

Stöchiometrie: TRIS : Ethylenoxid : Benzyl-glycidylether = 1 : 24 : 9

In einem 2l Autoklaven wird TRIS (50 g) in Toluol (130 ml) suspendiert und mit Kalium-*tert-*butylat (1,4 g) sowie Kronenether [18]Krone-6 (3,3 g) versetzt. Es wird dreimal mit N₂ gespült, ein Vordruck von ca. 1,3 bar N₂ eingestellt und die Temperatur auf 130°C erhöht. Ethylenoxid (84 g) wird innerhalb von 2 h zudosiert, wobei die Temperatur bei 130°C gehalten wird. Anschließend wird 5 h bei 130°C und dann über Nacht bei 40°C nachgerührt.

Am nächsten Tag wird das Reaktionsgemisch auf 120°C erhitzt und Benzylglycidylether (CAS. 2930-05-4; 117 g) wird über einen Zeitraum von 2 h zudosiert, wobei die Temperatur bei 120°C gehalten wird.
Anschließend wird 5 h bei 120°C und dann über Nacht bei 40°C nachgerührt. Flüchtige organische Verbindungen werden durch einen Stickstoffstrom entfernt und der Reaktor wird entleert.

Das basische Rohprodukt wird mit Hilfe von handelsüblichen Mg-Silikaten neutralisiert, welche anschließend abfiltriert werden. Das Lösungsmittel wird mit Hilfe eines Rotationsverdampfers entfernt und das Produkt wird anschließend für 3 h bei 80°C und p = 20 mbar nachgetrocknet. Man erhält 240 g des benzylgeschützen Zwischenproduktes. Das so erhaltene Zwischenprodukt (200 g) wird in einem Gemisch aus Ethylacetat (500 ml) und H₂O (500 ml) gelöst, mit Palladium auf Aktivkohle (10%, 20 g) versetzt und in einen 2,5 I Autoklaven überführt. Es wird dreimal mit N₂ inertisiert und anschließend Wasserstoff bis zu einem Druck von 10 bar zudosiert. Anschließend wird der Reaktorinhalt auf 80°C erhitzt und diese Reaktionstemperatur für 20 h gehalten, wobei der Wasserstoffdruck bei 10 bar gehalten wird. Der Reaktor wird auf Raumtemperatur abgekühlt, entspannt, dreimal mit N₂ inertisiert und entleert.
Der heterogene Katalysator wird mittels einer Drucknutsche unter N₂ abfiltriert und organische Lösungsmittel werden am Rotationsverdampfer unter Vakuum bei 80°C entfernt.

Man erhält 396,5 g des Produktes, das nach Karl-Fischer-Titration 65,8% Wasser enthält. Für analytische Zwecke wird bei einer kleinen Probe das Wasser azeotrop mit Toluol entfernt. SEC (THF + 0,1% Trifluoressigsäure als Elutionsmittel) zeigt Mn = 2090 g/mol und Mw = 2440 g/mol und eine Polydispersität Mw/Mn = 1.2.
Das Produkt wird auch mittels ¹H-NMR eindeutig charakterisiert.

### 1.8 Synthese von TRIS[(CH₂CH₂-O)₈-(-CH₂-CH(CH₂OH)-O)₂H]₃

Die Verbindung wurde durch Variation der Stöchiometrie (TRIS : Ethylenoxid : Benzylglycidylether = 1 : 24 : 6) analog zu Beispiel 1.7 hergestellt.

### 1.9 Synthese von TRIS[(CH₂CH₂-O)₁₁-(-CH₂-CH(CH₂OH)-O)₁H]₃

Die Verbindung wurde durch Variation der Stöchiometrie (TRIS : Ethylenoxid : Benzylglycidylether = 1 : 33 : 3) analog zu Beispiel 1.7 hergestellt.

### 1.10 Synthese von TRIS[(CH₂CH₂O)₇-(-CH₂-CH(CH₂OH)-O)₄H]₃

Die Verbindung wurde durch Variation der Stöchiometrie (TRIS : Ethylenoxid : Benzylglycidylether = 1 : 21 : 12) analog zu Beispiel 1.7 hergestellt.

### 1.11 Synthese von TRIS[(CH₂CH₂-O)₆-(-CH₂-CH(CH₂OH)-O)₄H]₃

Die Verbindung wurde durch Variation der Stöchiometrie (TRIS : Ethylenoxid : Benzylglycidylether = 1 : 18 : 12) analog zu Beispiel 1.7 hergestellt.

### 1.12 Synthese von TRIS[(CH₂CH₂-O)₅-(-CH₂-CH(CH₂OH)-O)₅H]₃

Die Verbindung wurde durch Variation der Stöchiometrie (TRIS : Ethylenoxid : Benzylglycidylether = 1 : 15 : 15) analog zu Beispiel 1.7 hergestellt.

### 1.13 Synthese von TRIS[(CH₂CH₂-O)₁₀H]₃

In einem 2l Autoklaven wird TRIS (50g) in Toluol (130 ml) suspendiert und mit Kalium-*tert-*butylat (1,4 g) sowie Kronenether [18]Krone-6 (3,3 g) versetzt. Es wird dreimal mit N₂ gespült, ein Vordruck von ca. 1.3 bar N₂ eingestellt und die Temperatur auf 130°C erhöht. Ethylenoxid (105 g) wird innerhalb von 2 h zudosiert, wobei die Temperatur bei 130°C gehalten wird. Anschließend wird 5 h bei 130°C und dann über Nacht bei 40°C nachgerührt. Flüchtige organische Verbindungen werden durch einen Stickstoffstrom entfernt und der Reaktor wird entleert. Das basische Rohprodukt wird mit Hilfe von handelsüblichen Mg-Silikaten neutralisiert, welche anschließend abfiltriert werden. Das Lösungsmittel wird mit Hilfe eines Rotationsverdampfers entfernt und das Produkt wird anschließend für 3 h bei 80°C und p = 20 mbar nachgetrocknet. Man erhält 155 g des Produktes mit einer OH-Zahl von 89,2 mg KOH/g. Das Produkt wird auch mittels ¹H-NMR charakterisiert.

### 1.14 Synthese von TRIS[(CH₂CH₂-O)₉-(-CH₂COONa)]₃

Ein Gemisch des Produktes aus Synthesebeispiel 1.13 (92,2 g) und Raney-Kupfer (18,44 g, wasserfeucht, ca. 12 g Trockengehalt) wird auf 200°C erhitzt. Bei dieser Temperatur wird Natronlauge (5%, 117,48 g) über einen Zeitraum von 6 h zudosiert, wobei Wasser und Wasserstoff kontinuierlich aus dem Reaktionsgemisch entweichen. Nach beendeter Zugabe wird das Reaktionsgemisch 0,5 h bei 200°C nachgerührt und danach auf Raumtemperatur abgekühlt. Das Reaktionsgemisch wird in Wasser aufgenommen und der heterogene Katalysator wird abfiltriert. Anschließend wird das Filtrat mit Hilfe eines Rotationsverdampfers eingeengt. Das Produkt wird mittels ¹H-NMR charakterisiert und der Wassergehalt mittels Karl-Fischer-Titration bestimmt (3,6%).

### 1.15 Synthese von TRIS[(CH₂CH₂-O)₉H]₃

In einem 0,3 I Autoklaven wird TRIS (25,2 g) in Toluol (46,4 g) suspendiert und mit Kalium-*tert*-butylat (0,84 g) versetzt.
Es wird dreimal mit N₂ gespült, ein Vordruck von ca. 1.3 bar N₂ eingestellt und die Temperatur auf 130°C erhöht. Ethylenoxid (47,52 g) wird zudosiert, wobei die Temperatur bei 130°C gehalten wird. Anschließend wird über Nacht 5 bei 130°C nachgerührt. Es wird auf 40°C abgekühlt, flüchtige organische Verbindungen werden durch einen Stickstoffstrom entfernt und der Reaktor wird entleert. Das basische Rohprodukt wird mit Hilfe von handelsüblichen Mg-Silikaten neutralisiert, welche anschließend abfiltriert werden. Das Lösungsmittel wird mit Hilfe eines Rotationsverdampfers entfernt und das Produkt wird anschließend für 3 h bei 80°C und p = 20 mbar nachgetrocknet. Man erhält 118 g des Produktes mit einer OH-Zahl von 100,2 mg KOH/g. Das Produkt wird auch mittels ¹H-NMR charakterisiert.

### 1.16 Synthese von TRIS[(CH₂CH₂-O)₉H]₃, Sulfatierungsgrad 50%

Das Produkt aus Synthesebeispiel 1.15 (50 g), Aminosulfonsäure (CAS 5329-14-6; 9,1 g) und katalytische Mengen Harnstoff (32 mg) werden für 7 h auf 85°C erhitzt, wobei sich eine hellbraune Masse bildet.
Nach Abkühlen auf Raumtemperatur werden 40 g Wasser zugegeben und das Reaktionsgemisch wird auf 80°C erhitzt. Der pH-Wert der so erhaltenen Lösung wird mit 50%-iger Natronlauge (5,6 g) auf pH = 9 eingestellt. Per ¹H-NMR wird der Sulfatierungsgrad bestimmt (50%). Der Wassergehalt der Lösung wird durch Karl-Fischer-Titration bestimmt (41,5%).

### 1.17 Synthese von TRIS[(CH₂CH₂-O)₆H]₃

Analog zu Synthesebeispiel 1.13 wird TRIS (100 g) mit Ethylenoxid (126 g) umgesetzt. Man erhält 223 g des Produktes.

### 1.18 Synthese von TRIS[(CH₂CH₂-O)₆-Glycosid]₃

Ein Gemisch des Produktes aus Synthesebeispiel 1.17 (11,3 g), Acetobrom-α-D-Glucose (13,1 g; CAS 572-09-8, Firma Aldrich), Kaliumcarbonat (5,45 g), Benzyltributylammoniumchlorid (0,49 g) und Chloroform (30 ml) wird für 21 h bei Raumtemperatur gerührt. Anschließend werden feste Bestandteile abfiltriert und es wird mit Chloroform (20 ml) nachgewaschen. Das Filtrat wird mit Hilfe eines Rotationsverdampfers eingeengt. Man erhält 23,7 g des acetylgeschützten Zwischenproduktes. Zur Entschützung wird das Zwischenprodukt (8,0 g) in Methanol (100 ml) gelöst und eine Lösung von NaOMe in MeOH (30%, 1,8 g) wird zugegeben. Die so erhaltene Lösung wird über Nacht bei Raumtemperatur gerührt, bevor der saure Ionentauscher Amberlite IRC86 (Firma Fluka, 10 g) zugegeben wird. Der Ionentauscher wird anschließend abfiltriert und das Filtrat wird mit Hilfe eines Rotationsverdampfers eingeengt. Man erhält das Produkt in Form einer dunklen Masse. MALDI-MS bestätigt die Glycosylierung.

### Beispiel 2 Physikalisch-chemische Untersuchungen

### Viskosität

Die Viskositätsmessungen wurden mit einem Rheometer (Anton Paar MCR 301) durchgeführt. Für die Viskositätsmessungen wurden wässrige Lösungen der Komponenten verwendet.

Zum Lösen der erfindungsgemäßen Tris(2-hydroxyphenyl)-methan-Derivate wurden die folgenden wässrigen Medien verwendet:

### Vollentsalztes Wasser (VE-Wasser)

### Lagerstättenwasser der Erdöl-Lagerstätte Bockstedt in Norddeutschland (synthetisch):

Gesamtsalinität: 185 548 mg/l
Na⁺ 52079 mg/l, Mg²⁺ 2681 mg/l, Ca²⁺ 15383 mg/l, Cl⁻ 115105 mg/l, Borat 117 mg/l, SO₄²⁻ 183 mg/l; Verhältnis Alkalimetallionen / Erdalkalimetallionen: 2,9; Lagerstätten-Wasser mit hohem Ca²⁺- Anteil.

Soweit nicht anders angegeben beziehen sich die Gehaltsangaben auf den Massenanteil in Gewichtsprozent.

Untersuchung von TRIS[(-CH₂-CH₂-O)₁₁H]₃ und den verschiedenen Tris(2-hydroxyphenyl)-methan-Derivaten

### Verdickende Eigenschaften

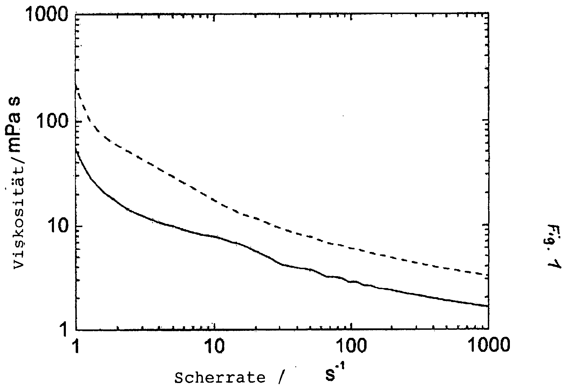

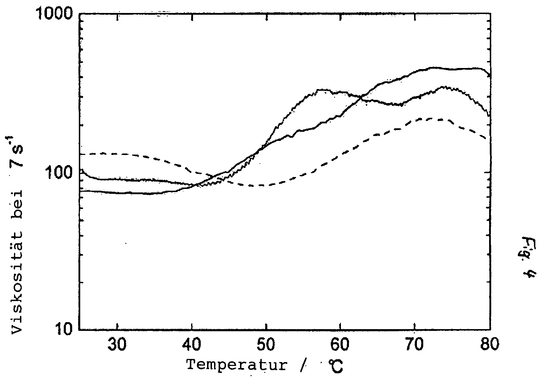

Die nachfolgenden Untersuchungen zeigen die verdickenden Eigenschaften der erfindungsgemäßen Strukturen in salzfreier Lösung und unter hoch-salinen Bedingungen sowie über einen breiten Temperaturbereich. Die Ergebnisse zeigen, dass die verdickenden Eigenschaften der erfindungsgemäßen Verbindungen robust gegenüber Salinität und Temperatur sind (Figuren 1 - 4).
**Figur 1** zeigt das allgemeine Scherprofil der erfindungsgemäßen Synthesebeispiele. Man sieht für 0,25 Gew.-% Lösungen von TRIS[(-CH₂-CH₂-O)₁₁H]₃ (Synthesebeispiel 1.2) bei 25°C, dass die verdickende Wirkung (Viskosität) nicht nur in Salz-freier Lösung (untere Kurve) sondern auch unter hochsalinen Bedingungen (obere Kurve: Lagerstättenwasser) unter Praxis-relevanten Scherbedingungen erhalten bleibt.

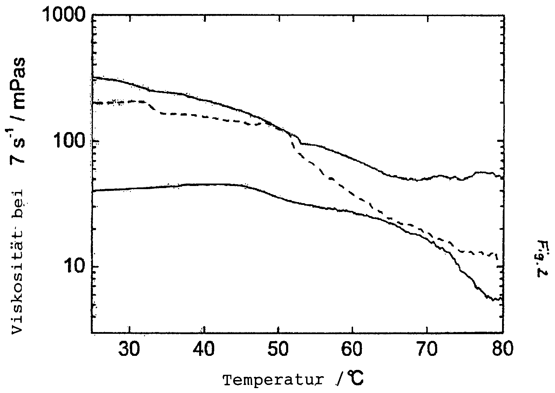

**Figur 2** zeigt die temperaturabhängige Verdickung (Viskosität) von 25° bis 80° C unter hochsalinen Bedingungen (Lagerstätten-Wasser) für drei verschiedene Tris(2-hydroxyphenyl)-methan-Derivate (jeweils 1 %-Lösungen) bei einer konstanten Schergeschwindigkeit von 7 s⁻¹:
   a) TRIS[(-CH₂-CH₂-O)₁₁-SO₃Y]₃ (Synthesebeispiel 1.4; unten liegende Kurve)
   b) TRIS[(CH₂CH₂-O)₉-(-CH₂COONa)]₃ (Synthesebeispiel 1.14; oben liegende Kurve)
   c) TRIS[(CH₂CH₂-O)₉H]₃, Sulfatierungsgrad 50%(Synthesebeispiel 1.16; in der Mitte liegende Kurve).

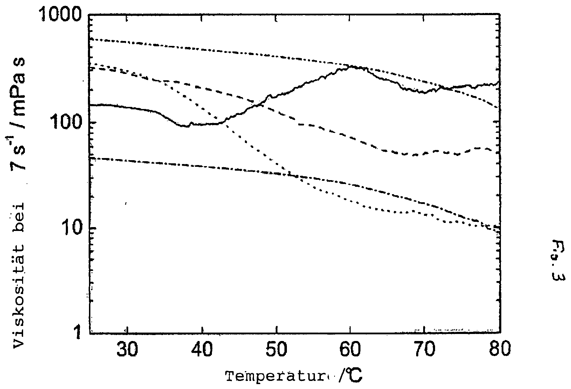

**Figur 3** zeigt die temperaturabhängige Verdickung (Viskosität) von 25° bis 80° C unter hochsalinen Bedingungen (Bockstedt Wasser) für zwei verschiedene Tris(2-hydroxyphenyl)-methan-Derivate (jeweils 1 %-Lösungen) im Vergleich zu drei bekannten Verdicker-Komponenten: Die polymeren Verdicker Floopaam 3630S (Firma SNF) sowie Alcoflood 1285 Red (Firma BASF) und das quartäre Ammoniumsalz Cetyltrimethylammoniumtosylat (CTAT, CAS 138-32-9; Firma Aldrich):
   a) TRIS[(CH₂CH₂-O)₉-(-CH₂COONa)]₃ (Synthesebeispiel 1.14(1%) (Linie - - - - - - -)
   b) Flopaam 3630S (1 %) (unterste Linie, _._._._._._._._.)
   c) Alcoflood 1285 Red (1%) (oberste Linie _.._.._.._.._.._.._.._..)
   d) CTAT (1%) (Linie .....................)
   e) TRiS[(CH₂CH₂-O)ₙ-(-CH₂-CH(CH₂OH)-O)₁H]₃ (Synthesebeispiel 1.9) (1%) (durchgezogene Linie).
**Figur 4** zeigt die temperaturabhängige Verdickung (Viskosität) von 25° bis 80° C bei einer konstanten Schergeschwindigkeit von 7 s⁻¹, unter hochsalinen Bedingungen (Lagerstättenwasser) für drei verschiedene Tris(2-hydroxyphenyl)-methan-Derivate (jeweils 1 %-Lösungen):
   a) TRIS[(CH₂CH₂-O)₁₁-(-CH₂-CH(CH₂OH)-O)₁H]₃ (Synthesebeispiel 1.9) (1%) (Linie .....................)
   b) TRIS[(CH₂CH₂-O)₈-(-CH₂-CH(CH₂OH)-O)₂H]₃ (Synthesebeispiel 1.8) (1%) (durchgezogene Linie)
   c) TRIS[(CH₂CH₂-O)₈-(-CH₂-CH(CH₂OH)-O)₃H]₃(Synthesebeispiel 1.7) (1%) (Linie------------).

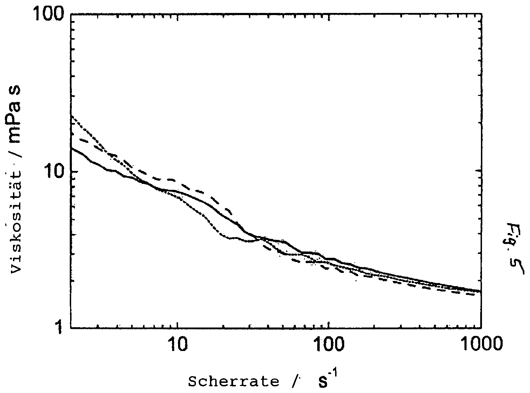

**Figur 5** zeigt die Stabilität der mizellaren Systeme mit TRIS[(-CH₂-CH₂-O)₁₁H]₃ (Synthesebeispiel 1.2) in salzhaltigem Wasser (93 g Salz/Liter; entspricht 50%-Salinität des Lagerstätten-Wassers), Wasser (0,1 %-Lösung des Verdickers) gegen Rohöl. Die Viskositäten wurden bei 25°C bestimmt:
   a) Ohne Kontakt zum Rohöl (durchgezogene Linie)
   b) 15 Tage nach Kontakt zum Rohöl (bei 50°C) (Linie - - - - - - - - -)
   c) 50 Tage nach Kontakt zum Rohöl (bei 25°C) (Linie ...............).

Die Messungen zeigen, dass sich die Viskosität der wässrigen Lösungen von TRIS[(-CH₂-CH₂-O)₁₁H]₃ (Synthesebeispiel 1.2) nach Kontakt mit Rohöl nicht ändert. Dies ist bei den bekannten viskoelastischen Lösungen von ionischen oder betainischen Tensiden, die Alkylketten als Hydrophob-Teil aufweisen (z.B. Cetylpyridiniumsalicylat) nicht zu beobachten.

### Viskoelastische Eigenschaften:

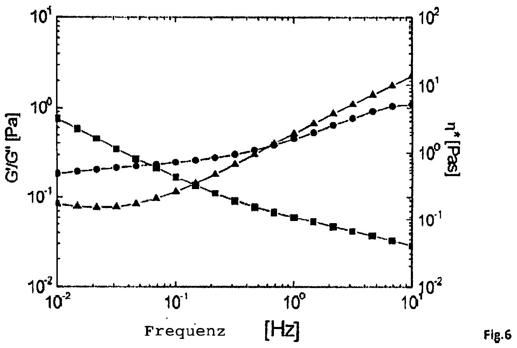

In oszillatorischen Scherexperimenten lassen sich die viskoelastischen Eigenschaften einer Flüssigkeit bestimmen. Der Speichermodul G' charakterisiert die elastischen Eigenschaften eines Materials, das Verlustmodul G" die viskosen Eigenschaften. Die Ergebnisse sind in Figur 6 gezeigt.
**Figur 6** zeigt die Eigenschaften (in Pa bzw. Pas):
   Speichermodul G' (-●-),
   Verlustmodul G" (-▲-) und
   komplexe Viskosität η* (-■-)
einer 0,25 %-Lösung in Lagerstätten-Wasser von TRIS[(-CH₂-CH₂-O)₁₁H]₃ (Synthesebeispiel 1.2) als Funktion der Frequenz (in Hz) bei konstanter Deformation von 10%.

### Grenzflächenaktivität:

Mittels der bekannten Du Nouy-Ringmethode (DIN EN 14210) wurde mit einem üblichen Tensiometer die Oberflächenspannung des Tensids in wässriger Lösung (vollentsalztes Wasser) bestimmt. Aus dem Verlauf der Oberflächenspannung als Funktion der TensidKonzentration wurde die kritische Mizellbildungs-Konzentration (cmc) bestimmt.

Für TRIS[(-CH₂-CH₂-O)₁₁H]₃ (Synthesebeispiel 1.2) beträgt die Oberflächenspannung 32 mN/m (1 g/l, 25°C). Die kritische Mizellbildungs-Konzentration in vollentsalztem Wasser ist 0,03 g/l (25°C).
Nach der Methode des hängenden Tropfens ("pendant drop") und des rotierenden Tropfens ("spinning drop") wurde die Grenzflächenspannung einer wässrigen Tensidlösung zu Hexadekan als Ölphase bestimmt. Die Tabelle zeigt die Grenzflächenspannungen (mN/m) von 0,5 %-Lösungen von TRIS[(-CH₂-CH₂-O)₁₁H]₃ (Synthesebeispiel 1.2) in salzhaltigem Wasser (93 g Salz/Liter; entspricht der 50%-igen Salinität des Lagerstättenwassers) gegen Rohöl der Lagerstätte Emlichheim gemessen unter den angegebenen Bedingungen.
Die Grenzflächenspannung des salzhaltigen Wassers gegen Rohöl ohne Additive beträgt 14 mN/m bei 23°C und 13 mN/m bei 60°C.

| Methode | 23°C | 60°C |
|---|---|---|
| Pendant drop (1h) | 2,21 | 0,73 |
| Spinning drop (1h) | 2,3 | 1,4 |

Die Messwerte zeigen, dass TRIS[(-CH₂-CH₂-O)₁₁H]₃ (Synthesebeispiel 1.2) die Grenzflächenspannung zwischen Rohöl und salzhaltigem Wasser erniedrigt, was wiederum die Mobilisierung des Öls erleichtert.

### Beispiel 3 Herstellung einer Formulierungen für die EOR

Die oben beschriebenen erfindungsgemäßen Triphenoxymethan-Derivate eignen sich besonders gut für Lagerstätten mit extremen Salinitäten (es wurde u. a . getestet bis zu Salzgehalt von ca. 200 g/l TDS) bei moderat erhöhten Temperaturen bis zu 80°C. Durch eine Verringerung der Salinität sind deutlich höhere Temperaturbereiche zugänglich. TriX ist besonders gut für Ca²⁺/Mg²⁺-haltige Lagerstätten geeignet.
Als viskoelastisches Tensidsystem weist TriX eine starke Scherverdünnung auf und kann deswegen in den erfindungsgemäßen Zusammensetzungen besonders gut gepumpt werden. Eine Degradation durch starke Scherung ist nicht zu beobachten. Die Möglichkeit schnellen Pumpens ist ein weiterer Vorteil gegenüber Polymer-Lösungen. Ein chemischer Abbau wird nicht beobachtet.

## Patentansprüche

1. Verfahren zur Erdölförderung, bei dem man eine wässrige Formulierung, die mindestens ein Derivat von Tris-(2-hydroxyphenyl)-methan der allgemeinen Formel (I) enthält, durch mindestens eine Injektionsbohrung in eine Erdöllagerstätte einpresst und der Lagerstätte durch mindestens eine Produktionsbohrung das Rohöl entnimmt, 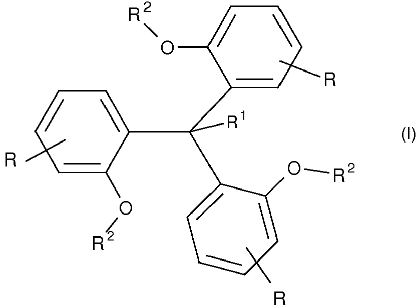 wobei in der Formel (I) die Reste R¹, R² und R die nachfolgende Bedeutung haben:
R: unabhängig voneinander jeweils 0 bis 4, vorzugsweise 1 bis 2, C₁- bis C₃₀-Kohlenwasserstoffreste pro Phenylring,
R¹ ein Rest ausgewählt aus der Gruppe von H, OH, F, Cl, Br, I sowie C₁- bis C₃₀-Kohlenwasserstoffgruppen, vorzugsweise H oder OH,
R² unabhängig voneinander Reste der allgemeinen Formel (III),
-(R⁵-O-)ₙ-R⁶-X (III)
wobei n für eine Zahl von 1 bis 50 steht, vorzugsweise 8 bis 35, und
wobei die Reste R⁵ unabhängig voneinander ausgewählt werden aus der Gruppe von Resten R⁷, R⁸ und R⁹, vorzugsweise aus der Gruppe R⁷ und R⁸, 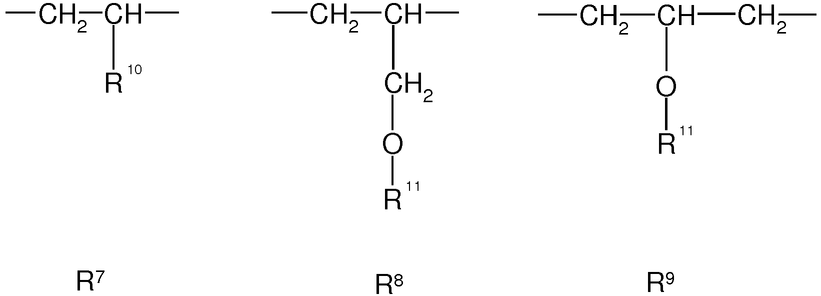 wobei R⁶, X, R¹⁰ und R¹¹ unabhängig voneinander die folgenden Bedeutungen haben:
R⁶ eine Einfachbindung oder eine Alkylengruppe mit 1 bis 10 Kohlenstoffatomen, welche optional funktionelle Gruppen als Substituenten aufweisen kann,
X H oder eine saure Gruppe oder ein Rest mit mindestens 2 OH-Gruppen oder eine Aminoxidgruppe,
R¹⁰ H oder ein C₁- bis C₆-Kohlenwasserstoffrest, vorzugsweise H oder Methyl
R¹¹ eine Gruppe -(R⁵-O-)ₘ-R⁶-X, wobei m für eine Zahl von 0 bis 50 steht,
und wobei die Gesamtzahl z aller Gruppen R⁵ in einem Rest R² 1 bis 50, vorzugsweise 8 bis 35, beträgt, mit der Maßgabe, dass es sich bei z um eine Zahl von 2 bis 50 handelt, falls mindestens ein X für H steht.

2. Verfahren zur Erdölförderung gemäß Anspruch 1, bei dem man eine wässrige Formulierung einsetzt, die mindestens ein Derivat von Tris-(2-hydroxyphenyl)-methan der allgemeinen Formel (II) enthält, 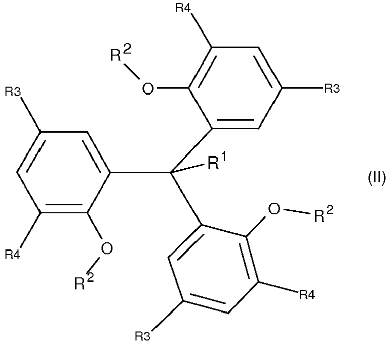 wobei R¹ und R² die in Anspruch 1 genannten Bedeutungen haben und R³ und R⁴ unabhängig voneinander für H oder einen C₁- bis C₃₀-Kohlenwasserstoffrest, insbesondere einen C₁- bis C₆-Kohlenwasserstoffrest, vorzugsweise t-Butylreste, stehen.

3. Verfahren gemäß Anspruch 2, **dadurch gekennzeichnet, dass** eine Verbindung der Formel (II) eingesetzt wird, in der alle R³ und R⁴ unabhängig voneinander geradkettige oder verzweigte aliphatische C₁- bis C₆- Kohlenwasserstoffreste sind, insbesondere t-Butylreste.

4. Verfahren gemäß einem der Ansprüche 1 bis 3 **dadurch gekennzeichnet, dass** die saure Gruppe ausgewählt wird aus Carboxylgruppen -COOM, Sulfonsäuregruppen - SO₃M, Sulfatgruppen -OSO₃M, Phosphonsäure-gruppen -PO₂M₂ oder Phosphorsäuregruppen -OPO₃M₂, wobei M für H⁺ oder ein einwertiges Gegenion, insbesondere Na-, K- oder Ammonium-Ionen, steht.

5. Verfahren gemäß einem der Ansprüche 1 bis 4, **dadurch gekennzeichnet, dass** es sich bei den Resten R⁵ um Gruppen R⁷ und/oder R⁸ handelt.

6. Verfahren gemäß einem der Ansprüche 1 bis 5, **dadurch gekennzeichnet, dass** die Temperatur der Erdöllagerstätte 10 bis 150°C, insbesondere 10 bis 120°C beträgt.

7. Verfahren gemäß einem der Ansprüche 1 bis 6, **dadurch gekennzeichnet, dass** die wässrige Formulierung als weitere Komponente mindestens ein Salz in einer Menge von 10.000 ppm bis 350.000 ppm enthält.

8. Verfahren gemäß einem der Ansprüche 1 bis 7, **dadurch gekennzeichnet, dass** die Erdölförderung aus Lagerstätten mit sehr hohem Salzgehalt und einer Lagerstätten-Temperatur von 10° bis 150°C erfolgt, wobei die Lagerstätte neben Erdöl auch Lagerstätten-Wasser mit einer Salinität von 20.000 ppm bis 350.000 ppm, insbesondere von 100.000 ppm bis 350.000 ppm umfasst, und das Erdöl eine Viskosität (gemessen bei Lagerstätten-Temperatur) von mindestens 3 mPa*s aufweist, indem man eine wässrige Formulierung umfassend mindestens ein Derivat von Tris-(2-hydroxyphenyl)-methan der Formel (I) durch mindestens eine Injektionsbohrung in die Erdöllagerstätte einpresst und der Lagerstätte durch mindestens eine Produktionsbohrung Rohöl entnimmt, wobei das Verfahren mindestens die folgenden Verfahrensschritte umfasst:
- Bereitstellen mindestens eines Tris-(2-hydroxyphenyl)-methan-Derivats der allgemeinen Formel (I) oder (II) als Rein-Substanz, Mischung oder als Konzentrat;
- Herstellen der wässrigen Formulierung der Tris-(2-hydroxyphenyl)-methan-Komponente(n) durch Verdünnen des gemäß Schritt (1) bereitgestellten Konzentrats (K) vor Ort mit Wasser auf eine Konzentration von 0,01 g/l bis 10 g/l, vorzugsweise sowie von 0,05 g/l bis 5 g/l,
- Injizieren der wässrigen Formulierung der Tris-(2-hydroxyphenyl)-methan-Komponente(n) in die Erdölformation, und
- Entnahme von Rohöl durch mindestens eine Produktionsbohrung.

9. Verwendung eines Tris(2-hydroxyphenyl)methan-Derivats der Formel (I) gemäß Anspruch 1 oder der Formel (II) gemäß Anspruch 2 als Verdicker, insbesondere als visco-elastischer Verdicker, in einer wässrigen Formulierung für die Erdölförderung.

10. Wässrige Formulierung, als Komponenten enthaltend:
0,001 bis 99 Gew. % mindestens eines Tris(2-hydroxyphenyl)methan-Derivats der Formel (I) gemäß Anspruch 1,
10 000 ppm bis 350 000 ppm anorganisches Salz, sowie ggf.
0,01 bis 5 Gew. % weiterer Hilfskomponenten für die Erdölförderung, sowie Wasser und ggf. weitere Lösungsmittel.

11. Wässrige Formulierung gemäß Anspruch 10, enthaltend:
0,1 bis 90 Gew. % eines Tris(2-hydroxyphenyl)methan-Derivats der Formel (II)
50 000 ppm bis 350 000 ppm anorganisches Salz, sowie
0,01 bis 5 Gew. % mindestens einer Hilfskomponente aus der Gruppe Tenside, Antioxidantien und Biozide, sowie Wasser und ggf. weitere Lösungsmittel.

12. Wässrige Formulierung gemäß Anspruch 10 oder 11, enthaltend:
0,1 bis 90 Gew. % eines Tris(2-hydroxyphenyl)methan-Derivats aus der Gruppe TRIS[(-CH₂-CH₂-O)₁₁H]₃
TRIS[(-CH₂-CH₂-O)₁₁H]₂[(-CH₂-CH₂-O)₁₁-SO₃Na]
TRIS[(-CH₂-CH₂-O)₁₁-SO₃Y]₃ (Y = Na⁺, NH₄⁺)
TRIS[(CH₂CH(CH₃)-O)_{4,4}-(-CH₂-CH₂-O)₁₃H]₃
TRIS mit 24 Glycidol-Einheiten und 9 EO-Einheiten
TRIS[(CH₂CH₂-O)₈-(-CH₂-CH(CH₂OH)-O)₃H]₃
TRIS[(CH₂CH₂-O)₈-(-CH₂-CH(CH₂OH)-O)₂H]₃
TRIS[(CH₂CH₂-O)₁₁-(-CH₂-CH(CH₂OH)-O)₁H]₃
TRIS[(CH₂CH₂-O)₇-(-CH₂-CH(CH₂OH)-O)₄H]₃
TRIS[(CH₂CH₂-O)₆-(-CH₂-CH(CH₂OH)-O)₄H]₃
TRIS[(CH₂CH₂-O)₅-(-CH₂-CH(CH₂OH)-O)₅H]₃
TRIS[(CH₂CH₂-O)₁₀H]₃
TRIS[(CH₂CH₂-O)₉-(-CH₂COONa)]₃
wobei TRIS für das Grundelement Tris-(3,5-di-*tert-*butyl-2-hydroxyphenyl)-methan steht,
50 000 ppm bis 350 000 ppm anorganisches Salz, sowie
0,01 bis 5 Gew. % mindestens einer Hilfskomponente aus der Gruppe Tenside, Antioxidantien und Biozide, sowie Wasser und ggf. weitere Lösungsmittel.

## Claims

1. A process for mineral oil production, in which an aqueous formulation comprising at least one derivative of tris(2-hydroxyphenyl)methane of the general formula (I) is injected into a mineral oil deposit through at least one injection well and the crude oil is withdrawn from the deposit through at least one production well, 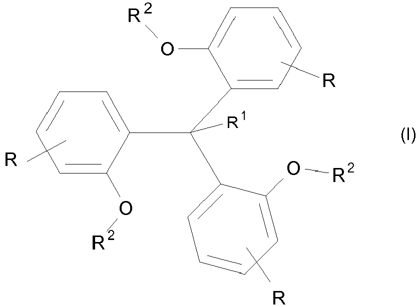 where the R¹, R² and R radicals in the formula (I) are each defined as follows:
R is in each case independently 0 to 4, preferably 1 to 2,
C₁- to C₃₀-hydrocarbyl radicals per phenyl ring,
R¹ is a radical selected from the group of H, OH, F, Cl, Br, I
and C₁- to C₃₀-hydrocarbyl groups, preferably H or OH,
R² are each independently radicals of the general formula (III),
-(R⁵-O-)ₙ-R⁶-X (III)
where n is a number from 1 to 50, preferably 8 to 35, and
where the R⁵ radicals are each independently selected from the group of R⁷, R⁸ and R⁹ radicals, preferably from the group of R⁷ and R⁸, 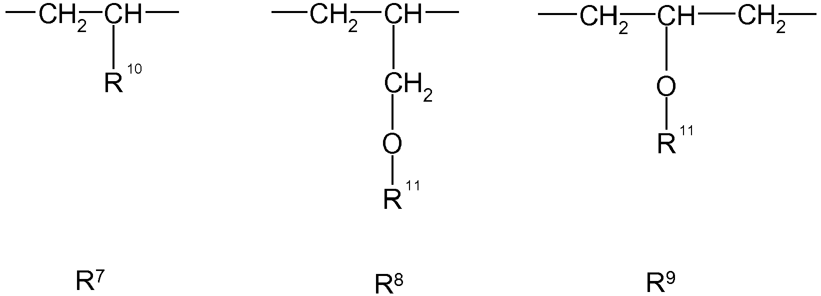 where R⁶, X, R¹⁰ and R¹¹ are each independently defined as follows:
R⁶ is a single bond or an alkylene group having 1 to 10 carbon atoms, which may optionally have functional groups as substituents,
X is H or an acidic group or a radical having at least 2 OH groups or an amine oxide group,
R¹⁰ is H or a C₁- to C₆-hydrocarbyl radical, preferably H or methyl,
R¹¹ is a -(R⁵-O-)ₘ-R⁶-X group, where m is a number from 0 to 50,
and where the total number z of all R⁵ groups in one R² radical is 1 to 50, preferably 8 to 35, with the proviso that z is a number from 2 to 50 if at least one X is H.

2. The process for mineral oil production according to claim 1, in which the aqueous formulation used comprises at least one derivative of tris(2-hydroxyphenyl)methane of the general formula (II) 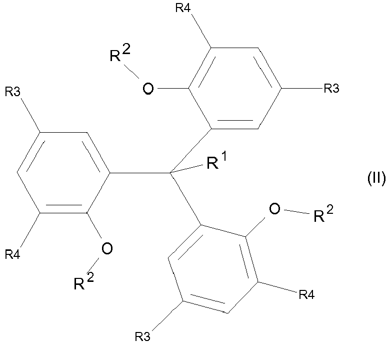 where R¹ and R² are each as defined in claim 1 and R³ and R⁴ are each independently H or a C₁- to C₃₀-hydrocarbyl radical, especially a C₁- to C₆-hydrocarbyl radical, preferably t-butyl radicals.

3. The process according to claim 2, wherein the compound of the formula (II) used is one in which all R³ and R⁴ are independently straight-chain or branched C₁- to C₆-hydrocarbyl radicals, especially t-butyl radicals.

4. The process according to any of claims 1 to 3, wherein the acid group is selected from carboxyl groups -COOM, sulfo groups -SO₃M, sulfate groups -OSO₃M, phosphonic acid groups -PO₂M₂ or phosphoric acid groups -OPO₃M₂, where M is H⁺ or a monovalent counterion, especially Na, K or ammonium ions.

5. The process according to any of claims 1 to 4, wherein the R⁵ radicals are R⁷ and/or R⁸ groups.

6. The process according to any of claims 1 to 5, wherein the temperature of the mineral oil deposit is 10 to 150°C, especially 10 to 120°C.

7. The process according to any of claims 1 to 6, wherein the aqueous formulation comprises, as a further component, at least one salt in an amount of 10 000 ppm to 350 000 ppm.

8. The process according to any of claims 1 to 7, wherein the mineral oil production is effected from deposits with a very high salt content and a deposit temperature of 10 to 150°C, said deposit comprising, as well as mineral oil, also deposit water with a salinity of 20 000 ppm to 350 000 ppm, especially of 100 000 ppm to 350 000 ppm, and the mineral oil having a viscosity (measured at deposit temperature) of at least 3 mPa*s, by injecting an aqueous formulation comprising at least one derivative of tris(2-hydroxyphenyl)methane of the formula (I) into the mineral oil deposit through at least one injection well and withdrawing crude oil from the deposit through at least one production well, said process comprising at least the following process steps:
- providing at least one tris(2-hydroxyphenyl)methane derivative of the general formula (I) or (II) as a pure substance, mixture or concentrate;
- preparing the aqueous formulation of the tris(2-hydroxyphenyl)methane component(s) by diluting the concentrate (K) provided in step (1) on site with water to a concentration of 0.01 g/l to 10 g/l, preferably of 0.05 g/l to 5 g/l,
- injecting the aqueous formulation of the tris(2-hydroxyphenyl)methane component(s) into the mineral oil formation, and
- withdrawing crude oil through at least one production well.

9. The use of a tris(2-hydroxyphenyl)methane derivative of the formula (I) according to claim 1 or of the formula (II) according to claim 2 as a thickener, especially as a viscoelastic thickener, in an aqueous formulation for mineral oil production.

10. An aqueous formulation comprising, as components:
0.001 to 99% by weight of at least one tris(2-hydroxyphenyl)methane derivative of the formula (I) according to claim 1,
10 000 ppm to 350 000 ppm of inorganic salt, and optionally
0.01 to 5% by weight of further auxiliary components for mineral oil production, and water and optionally further solvents.

11. The aqueous formulation according to claim 10, comprising:
0.1 to 90% by weight of a tris(2-hydroxyphenyl)methane derivative of the formula (II)
50 000 ppm to 350 000 ppm of inorganic salt, and
0.01 to 5% by weight of at least one auxiliary component from the group of surfactants, antioxidants and biocides, and water and optionally further solvents.

12. The aqueous formulation according to claim 10 or 11, comprising:
0.1 to 90% by weight of a tris(2-hydroxyphenyl)methane derivative from the group of
TRIS[(-CH₂-CH₂-O)₁₁H]₃
TRIS[(-CH₂-CH₂-O)₁₁H]₂[(-CH₂-CH₂-O)₁₁-SO₃Na]
TRIS[(-CH₂-CH₂-O)₁₁-SO₃Y]₃ (Y = Na⁺, NH₄⁺)
TRIS[(CH₂CH(CH₃)-O)_{4,4}-(-CH₂-CH₂-O)₁₃H]₃
TRIS with 24 glycidol units and 9 EO units
TRIS[(CH₂CH₂-O)₈-(-CH₂-CH(CH₂OH)-O)₃H]₃
TRIS[(CH₂CH₂-O)₈-(-CH₂-CH(CH₂OH)-O)₂H]₃
TRIS[(CH₂CH₂-O)₁₁-(-CH₂-CH(CH₂OH)-O)₁H]₃
TRIS[(CH₂CH₂-O)₇-(-CH₂-CH(CH₂OH)-O)₄H]₃
TRIS[(CH₂CH₂-O)₆-(-CH₂-CH(CH₂OH)-O)₄H]₃
TRIS[(CH₂CH₂-O)₅-(-CH₂-CH(CH₂OH)-O)₅H]₃
TRIS[(CH₂CH₂-O)₁₀H]₃
TRIS[(CH₂CH₂-O)₉-(-CH₂COONa)]₃
where TRIS represents the tris(3,5-di-*tert*-butyl-2-hydroxyphenyl)methane base element,
50 000 ppm to 350 000 ppm of inorganic salt, and
0.01 to 5% by weight of at least one auxiliary component from the group of surfactants, antioxidants and biocides, and water and optionally further solvents.

## Revendications

1. Procédé d'extraction de pétrole, selon lequel une formulation aqueuse qui contient au moins un dérivé de tris-(2-hydroxyphényl)-méthane de formule générale (I) est injectée par au moins un forage d'injection dans un gisement de pétrole et le pétrole brut est extrait du gisement par au moins un forage de production, 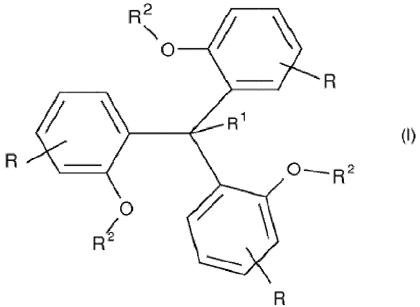 dans la formule (I), les radicaux R¹, R² et R ayant la signification suivante :
les R chacun indépendamment les uns des autres, 0 à 4, de préférence 1 à 2, radicaux hydrocarbonés en C₁ à C₃₀ par cycle phényle,
R¹ un radical choisi dans le groupe constitué par H, OH, F, Cl, Br, I, ainsi que les groupes hydrocarbonés en C₁ à C₃₀, de préférence H ou OH,
les R² indépendamment les uns des autres, des radicaux de formule générale (III)
-(R⁵-O-)ₙ-R⁶-X (III)
dans laquelle n représente un nombre de 1 à 50, de préférence 8 à 35, et
dans laquelle les radicaux R⁵ sont choisis indépendamment les uns des autres dans le groupe constitué par les radicaux R⁷, R⁸ et R⁹, de préférence dans le groupe constitué par R⁷ et R⁸, 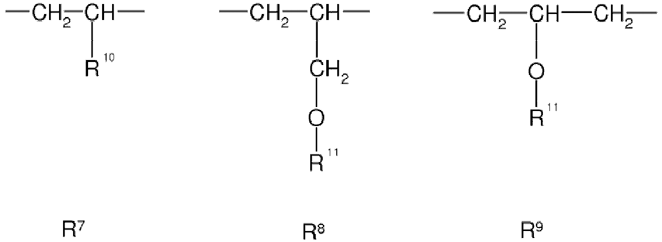
dans lesquels R⁶, X, R¹⁰ et R¹¹ ont indépendamment les uns des autres les significations suivantes :
R⁶ une simple liaison ou un groupe alkylène de 1 à 10 atomes de carbone, qui peut éventuellement comprendre des groupes fonctionnels en tant que substituants,
X H ou un groupe acide ou un radical contenant au moins 2 groupes OH ou un groupe aminoxyde,
R¹⁰ H ou un radical hydrocarboné en C₁ à C₆, de préférence H ou méthyle,
R¹¹ un groupe -(R⁵-O-)ₘ-R⁶-X, m représentant un nombre de 0 à 50,
et le nombre total z de tous les groupes R⁵ dans un radical R² étant de 1 à 50, de préférence de 8 à 35, à condition que z soit un nombre de 2 à 50 si au moins un X représente H.

2. Procédé d'extraction de pétrole selon la revendication 1, selon lequel une formulation aqueuse est utilisée, qui contient au moins un dérivé de tris-(2-hydroxyphényl)-méthane de formule générale (II) 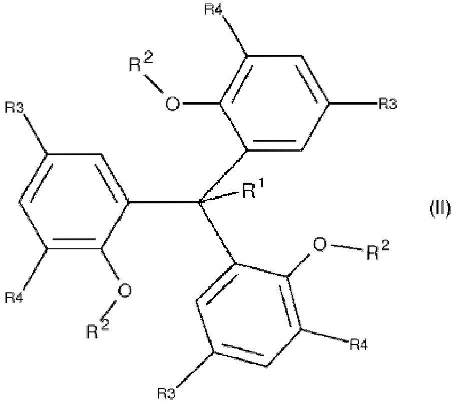 dans laquelle R¹ et R² ont les significations indiquées dans la revendication 1, et R³ et R⁴ représentent indépendamment l'un de l'autre H ou un radical hydrocarboné en C₁ à C₃₀, notamment un radical hydrocarboné en C₁ à C₆, de préférence des radicaux t-butyle.

3. Procédé selon la revendication 2, **caractérisé en ce qu'**un composé de formule (II) est utilisé, dans lequel tous les R³ et R⁴ sont indépendamment les uns des autres des radicaux hydrocarbonés en C₁ à C₆ aliphatiques linéaires ou ramifiés, notamment des radicaux t-butyle.

4. Procédé selon l'une quelconque des revendications 1 à 3, **caractérisé en ce que** le groupe acide est choisi parmi les groupes carboxyle -COOM, les groupes acide sulfonique -SO₃M, les groupes sulfate -OSO₃M, les groupes acide phosphonique -PO₂M₂ ou les groupes acide phosphorique -OPO₃M₂, M représentant H⁺ ou un contre-ion monovalent, notamment des ions Na, K ou ammonium.

5. Procédé selon l'une quelconque des revendications 1 à 4, **caractérisé en ce que** les radicaux R⁵ sont des groupes R⁷ et/ou R⁸.

6. Procédé selon l'une quelconque des revendications 1 à 5, **caractérisé en ce que** la température du gisement de pétrole est de 10 à 150 °C, notamment de 10 à 120 °C.

7. Procédé selon l'une quelconque des revendications 1 à 6, **caractérisé en ce que** la formulation aqueuse contient en tant que composant supplémentaire au moins un sel en une quantité de 10 000 ppm à 350 000 ppm.

8. Procédé selon l'une quelconque des revendications 1 à 7, **caractérisé en ce que** l'extraction de pétrole a lieu à partir de gisements ayant une teneur en sels très élevée et une température de gisement de 10 à 150 °C, le gisement comprenant en plus du pétrole également de l'eau de gisement ayant une salinité de 20 000 ppm à 350 000 ppm, notamment de 100 000 ppm à 350 000 ppm, et le pétrole présentant une viscosité (mesurée à la température du gisement) d'au moins 3 mPa*s, selon lequel une formulation aqueuse comprenant au moins un dérivé de tris-(2-hydroxyphényl)-méthane de formule générale (I) est injectée par au moins un forage d'injection dans le gisement de pétrole et le pétrole brut est extrait du gisement par au moins un forage de production, le procédé comprenant au moins les étapes de procédé suivantes :
- la préparation d'au moins un dérivé de tris-(2-hydroxyphényl)-méthane de formule générale (I) ou (II) en tant que substance pure, mélange ou en tant que concentré ;
- la fabrication de la formulation aqueuse du ou des composants tris-(2-hydroxyphényl)-méthane par dilution du concentré (K) préparé selon l'étape (1) sur place avec de l'eau à une concentration de 0,01 g/l à 10 g/l, de préférence de 0,05 g/l à 5 g/l,
- l'injection de la formulation aqueuse du ou des composants tris-(2-hydroxyphényl)-méthane dans la formation pétrolifère, et
- l'extraction de pétrole brut par au moins un forage de production.

9. Utilisation d'un dérivé de tris-(2-hydroxyphényl)-méthane de formule (I) selon la revendication 1 ou de formule (II) selon la revendication 2 en tant qu'épaississant, notamment en tant qu'épaississant viscoélastique, dans une formulation aqueuse pour l'extraction de pétrole.

10. Formulation aqueuse, contenant en tant que composants :
0,001 à 99 % en poids d'au moins un dérivé de tris-(2-hydroxyphényl)-méthane de formule (I) selon la revendication 1,
10 000 ppm à 350 000 ppm d'un sel inorganique, et éventuellement
0,01 à 5 % en poids d'autres composants auxiliaires pour l'extraction de pétrole, ainsi que de l'eau et éventuellement d'autres solvants.

11. Formulation aqueuse selon la revendication 10, contenant :
0,1 à 90 % en poids d'un dérivé de tris-(2-hydroxyphényl)-méthane de formule (II),
50 000 ppm à 350 000 ppm d'un sel inorganique, et
0,01 à 5 % en poids d'au moins un composant auxiliaire du groupe constitué par les tensioactifs, les antioxydants et les biocides, ainsi que de l'eau et éventuellement d'autres solvants.

12. Formulation aqueuse selon la revendication 10 ou 11, contenant :
0,1 à 90 % en poids d'un dérivé de tris-(2-hydroxyphényl)-méthane du groupe constitué par :
TRIS[(-CH₂-CH₂-O)₁₁H]₃
TRIS[(-CH₂-CH₂-O)₁₁H]₂[(-CH₂-CH₂-O)₁₁-SO₃Na]
TRIS[(-CH₂-CH₂-O)₁₁-SO₃Y]₃ (Y = Na⁺, NH₄⁺)
TRIS[(CH₂CH(CH₃)-O)_{4,4}-(-CH₂-CH₂-O)₁₃H]₃
TRIS avec 24 unités glycidol et 9 unités EO TRIS[(CH₂CH₂-O)₈-(-CH₂-CH(CH₂OH)-O)₃H]₃
TRIS[(CH₂CH₂-O)₈-(-CH₂-CH(CH₂OH)-O)₂H]₃
TRIS[(CH₂CH₂-O)₁₁-(-CH₂-CH(CH₂OH)-O)₁H]₃
TRIS[(CH₂CH₂-O)₇-(-CH₂-CH(CH₂OH)-O)₄H]₃
TRIS[(CH₂CH₂-O)₆-(-CH₂-CH(CH₂OH)-O)₄H]₃
TRIS[(CH₂CH₂-O)₅-(-CH₂-CH(CH₂OH)-O)₅H]₃
TRIS[(CH₂CH₂-O)₁₀H]₃
TRIS[(CH₂CH₂-O)₉-(-CH₂COONa)]₃
TRIS représentant l'élément de base tris-(3,5-di-tert-butyl-2-hydroxyphényl)-méthane,
50 000 ppm à 350 000 ppm d'un sel inorganique, et
0,01 à 5 % en poids d'au moins un composant auxiliaire du groupe constitué par les tensioactifs, les antioxydants et les biocides, ainsi que de l'eau et éventuellement d'autres solvants.
